# EUROPEAN PATENT SPECIFICATION

(11) **EP 1 687 631 B1**
(45) Date of publication and mention of the grant of the patent: **29.04.2009**
(21) Application number: 04768604.3
(22) Date of filing: 27.09.2004
(51) Int. Cl.: G01N 33/50, A61P 35/00, A61P 9/10, A61P 3/10, A61P 25/00

(54) **METHODS FOR IDENTIFYING COMPOUNDS INVOLVED IN THE PHOSPHOINOSITIDE-MEDIATED SIGNALLING AND USE THEREOF IN THE MANUFACTURE OF MEDICAMENTS**
VERFAHREN ZUR IDENTIFIZIERUNG VON AN DER PHOSPHOINOSITID-VERMITTELTEN SIGNALGEBUNG BETEILIGTEN VERBINDUNGEN SOWIE VERWENDUNG DAVON BEI DER HERSTELLUNG VON ARZNEIMITTELN
PROCEDES D'IDENTIFICATION DE COMPOSES CHIMIQUES IMPLIQUES DANS LA SIGNALISATION MEDIEE PAR PHOSPHOINOSITIDE ET LEUR UTILISATION DANS LA FABRICATION DE MEDICAMENTS

(30) Priority: 27.09.2003 GB 0322689
(43) Date of publication of application: 09.08.2006
(73) Proprietor: MEDICAL RESEARCH COUNCIL, London W1N 4AL (GB)
(72) Inventor: KIMBER, Wendy, A. Cambridge Antibody Technology, Cambridge, CB1 6GH (GB); ALESSI, Dario, R. MRC Protein Phosphorylation Unit, Dundee DD1 5EH (GB)
(74) Representative: Pilkington, Stephanie Joan
(86) International application number: PCT/GB2004/004060
(87) International publication number: WO 2005/031347

(56) References cited:
- WO-A-01/57240
- WO-A-02/12276
- WO-A-03/011901
- US-A- 5 821 075
- BOMPARD GUILLAUME ET AL: "Membrane targeting of protein tyrosine phosphatase PTPL1 through its FERM domain via binding to phosphatidylinositol 4,5-biphosphate." JOURNAL OF CELL SCIENCE, vol. 116, no. 12, 15 June 2003 (2003-06-15), pages 2519-2530, XP002319141 ISSN: 0021-9533
- KIMBER WENDY A ET AL: "Interaction of the protein tyrosine phosphatase PTPL1 with the PtdIns(3,4)P2-binding adaptor protein TAPP1." BIOCHEMICAL JOURNAL, vol. 376, no. 2, 1 December 2003 (2003-12-01), pages 525-535, XP002319142 ISSN: 0264-6021
- KIMBER W A ET AL: "Evidence that the tandem-pleckstrin-homology-domain-containi ng protein TAPP1 interacts with Ptd(3,4)P2 and the multi-PDZ-domain-containing protein MUPP1 in vivo" BIOCHEMICAL JOURNAL 01 FEB 2002 UNITED KINGDOM, vol. 361, no. 3, 1 February 2002 (2002-02-01), pages 525-536, XP001057247 ISSN: 0264-6021
- DOWLER S ET AL: "Identification of pleckstrin-homology-domain-containing proteins with novel phosphoinositide-binding specificities" BIOCHEMICAL JOURNAL 01 OCT 2000 UNITED KINGDOM, vol. 351, no. 1, 1 October 2000 (2000-10-01), pages 19-31, XP001096514 ISSN: 0264-6021
- MARSHALL AARON J ET AL: "TAPP1 and TAPP2 are targets of phosphatidylinositol 3-kinase signaling in B cells: Sustained plasma membrane recruitment triggered by the B-cell antigen receptor" MOLECULAR AND CELLULAR BIOLOGY, vol. 22, no. 15, August 2002 (2002-08), pages 5479-5491, XP002319143 ISSN: 0270-7306
- BOMPARD ET AL: "Protein-tyrosine Phosphatase PTPL1/FAP-1 Triggers Apoptosis in Human Breast Cancer cells" JOURNAL OF BIOLOGICAL CHEMISTRY, vol. 277, no. 49, 2002, pages 47861-47869,

## Description

The present invention relates to medicaments for the treatment of conditions in which phosphoinositide-mediated signalling is involved, including diabetes and ischaemic disease, for example stroke; and screening methods for identifying compounds useful in such medicaments.

Insulin and growth factors mediate many of their cellular effects through activation of phosphoinositide 3-kinase (PI 3-kinase), which phosphorylates phosphatidylinositol 4,5 bisphosphate (PtdIns(4,5)P₂) to generate the second messenger PtdIns(3,4,5)P₃ [1]. PtdIns(3,4,5)P₃ triggers the activation of downstream signalling pathways by recruiting signalling proteins containing pleckstrin homology (PH) domains to the plasma membrane [2].

As reviewed in, for example, [36], the insulin receptor is a transmembrane receptor comprising two extracellular α subunits and two transmembrane β subunits. On insulin binding, the receptor undergoes autophosphorylation on several tyrosine residues located on the cytoplasmic portion of the β subunits. Signalling molecules containing SH2 (Src homology 2) or PTB (phosphotyrosine binding) domains are recruited by the phosphorylated receptor. Most of the recruited proteins are members of the insulin receptor substrate (IRS) family, and include Shc (involved in the activation of the mitogen activated protein kinase (MAPK) pathway) and IRS-1 (involved in both metabolic and mitogenic signalling). IRS-1, for example, becomes heavily tyrosine phosphorylated following recruitment, and recruits several further SH2-containing proteins, including the p85 regulatory subunit of phosphatidylinositol 3-kinase (PI3-kinase). The p85 subunit recruits the p110 catalytic subunit of PI3-kinase, resulting in the activation of the PI3-kinase pathway.

PtdIns(3,4,5)P₃ is metabolised in two ways. It can either be dephosphorylated at the 3 position by the PTEN phosphatase to generate PtdIns(4,5)P₂ or at the 5 position by the SHIP1 and SHIP2 phosphatases to generate PtdIns(3,4)P₂ [3]. It has been suggested that PtdIns(3,4)P₂ may function as a signalling lipid as its cellular concentrations are low in unstimulated cells and markedly elevated after activation of PI 3-kinase [1, 4, 5]. Moreover in certain cellular systems, such as an integrin-signalling pathway in platelets, PtdIns(3,4)P₂ can be generated independently from PtdIns(3,4,5)P₃, by phosphorylation of PtdIns 3P catalysed by a PI 4-kinase [6].

Several PH domain-containing proteins such as PKB [7], PDK1 [7-9] and DAPP1 [10], bind PtdIns(3,4,5)P₃ and PtdIns(3,4)P₂ with similar affinity. Structural studies of the PH domains of PKB [11] and DAPP1 [12], demonstrate that basic residues on these proteins form specific interactions with the 3 and 4 inositol phosphate groups but the 5 phosphate is solvent exposed and does not bind residues on the PH domain. To date only the TAndem PH domain-containing Protein-1 (TAPP1), TAPP2 [13] and the PX domain of the phagocytic p47(phox) cytosolic subunit required for activation of NADPH oxidase, possess the properties of binding PtdIns(3,4)P₂ specifically [14, 15]. However, it should be noted that TAPP1 possesses over 1000-fold higher affinity for PtdIns(3,4)P₂ than does the PX domain of p47(phox) [15].

TAPP1 and TAPP2 are putative adaptor proteins consisting of two sequential PH domains in which the C-terminal PH domain binds PtdIns(3,4)P₂. The N-terminal PH domain does not interact with any lipid tested [13]. The structure of the C-terminal PH domain of TAPP1 coupled with mutagenesis studies suggested that basic residues interacted with the 3 and the 4 phosphate groups of PtdIns(3,4)P₂. Binding of TAPP1 to PtdIns(3,4,5)P₃ is likely to be sterically hindered by an Ala residue being located close to the position in which the 5 phosphate would be expected to reside [16]. Consistent with the notion that TAPP1 interacts with PtdIns(3,4)P₂ *in vivo*, we demonstrated that TAPP1 relocated from the cytosol to the plasma membrane of cells following stimulation with agonists that induced PtdIns(3,4)P₂, but not with those that mainly induced PtdIns(3,4,5)P₃ [17]. Similarly in B cells, both TAPP1 and TAPP2 were shown to translocate to the plasma membrane in response to antigen stimulation and this correlated with the formation of PtdIns(3,4)P₂ rather than production of PtdIns(3,4,5)P₃ [18]. Similar results have also been obtained in T-cell lines [19]. Although we have been unable to detect significant interaction of TAPP1 with PtdIns(3,4,5)P₃, another group has provided evidence that in vitro TAPP1 may interact with PtdIns(3,4,5)P₃ albeit more weakly than with PtdIns(3,4)P₂ [12].

TAPP1 and TAPP2, and screening methods relating thereto are described in WO 02/12276. The structure of the TAPP phosphoinositide-binding domain and mutants and screening methods relating thereto are described in WO 03/011901.

Stimulation of cells with insulin and growth factors frequently results in a transient production of PtdIns(3,4,5)P₃, whose levels peak after a few minutes and decline thereafter [1, 5]. The mechanism by which PtdIns(3,4,5)P3 declines after prolonged insulin and growth factor stimulation is poorly defined. We considered that one possibility is that PtdIns(3,4)P₂ which is generated after PtdIns(3,4,5)P₃ production, could serve as a specific negative feedback signal, recruiting to the plasma membrane PtdIns(3,4,5)P₃ phosphatases and/or protein tyrosine phosphatases. The latter could dephosphorylate receptors and adaptor subunits leading to inactivation of PI 3-kinase and/a reduction in the level of PtdIns(3,4,5)P₃. We provide evidence that supports this mechanism, by identifying protein tyrosine phosphatase L1 (PTPL1) as an enzyme that binds specifically to TAPP1 and TAPP2. We further provide methods for modulating phosphoinositide signalling and screening methods for identifying compounds useful for modulating phosphoinositide signalling.

A first aspect of the invention provides a method for identifying a compound for modulating the cellular activity or location of PTPL1, comprising the step of identifying a compound that modulates the interaction of PTPL1 with TAPP; or which modulates or mimics the interaction of TAPP with PtdIns(3,4)P₂; or which modulates the cellular location or interactions of TAPP.

The method may make use of a method for selecting or designing a compound for modulating the activity of Tandem PH domain containing Protein (TAPP) as described in WO 03/011901, for example may comprise the step of using molecular modelling means to select or design a compound that is predicted to interact with the phosphoinositide binding domain of TAPP, wherein a three-dimensional structure of at least a part of the phosphoinoisitide binding domain of the TAPP is compared with a three-dimensional structure of a compound, and a compound that is predicted to interact with the said phosphoinositide binding domain is selected.

It is considered that binding of PtdIns(3,4)P₂ to TAPP leads to translocation of TAPP, for example from the cytoplasm to the plasma membrane, and translocation of TAPP-bound PTPL1, for example from the cytoplasm to the plasma membrane. Binding of PtdIns(3,4)P₂ to TAPP is not considered significantly to change the binding of TAPP to PTPL1.

A compound which binds to the phosphoinositide binding domain of TAPP may inhibit the binding of PtdIns(3,4)P₂ to TAPP, or may mimic the binding of PtdIns(3,4)P₂ to TAPP, thereby modulating the translocation of TAPP and TAPP-bound PTPL1, for example from the cytoplasm to the plasma membrane..

Thus, the compound may be capable of affecting the intracellular location of TAPP and PTPL1; for example, it may inhibit or promote the translocation of the polypeptides to a membrane, for example the plasma membrane or golgi, vacuole, lysosome or endosome membrane.

Preferences in relation to this embodiment of the method of the first aspect of the invention are as indicated in WO 03/011901,. For example, it is preferred that the three-dimensional structure of at least a part of the phosphoinositide binding domain of the TAPP is a three-dimensional structure of at least a part of the phosphoinositide binding site of the TAPP and a compound that is predicted to interact with the said phosphoinositide binding site is selected. Alternatively, the compound may bind to a portion of the TAPP polypeptide that is not the binding site so as to interfere with the binding of the TAPP polypeptide to the phosphoinositide. In a still further example, the compound may bind to a portion of TAPP so as to decrease TAPP's phosphoinositide binding activity by an allosteric effect. This allosteric effect may be an allosteric effect that is involved in the natural regulation of the said polypeptide's activity.

By the term Tandem Pleckstrin Homolgy domain containing protein (TAPP) is included the polypeptides termed mammalian (for example human or mouse) TAPP1 and TAPP2 identified in Dowler et al (2000) Biochem J 351, 19-31 and in WO 02/12276.

In relation to polypeptides or structures used in screening methods or assays the term also includes fragments and fusions thereof that comprise the C-terminal PH domain, as discussed in WO 03/011901, for example in Example 1. A particularly preferred TAPP polypeptide is the C-terminal PH domain of TAPP1, optionally with an N-terminal GST tag, as described in Example 1 of WO 03/011901. Further examples are considered to include fragments, variants, derivatives or fusions thereof, or fusions of fragments, variants or derivatives, that retain the phosphoinositide binding properties of full length TAPP1 or TAPP2, or the C-terminal PH domain either thereof, as discussed further below (ie that are capable of binding to PtdIns(3,4)P₂).

Alternatively or in addition, the TAPP polypeptide preferably retains the portion considered to be involved in binding to PTPL1, for example the C-terminal at least three amino acids, which correspond to the minimal sequence motif (Ser/Thr-Xaa-Val/Ile (Kornau et al (1995) Science 269, 1737-1740; Sogyang et al (1997) Science 275, 73-77)) required for binding to a PDZ domain. The TAPP polypeptide preferably retains the PTPL1-binding properties of TAPP1 or TAPP2.

The N-terminal PH domain of TAPP may also be involved in interactions, for example with other polypeptides or cellular structures; accordingly it may be useful for the TAPP polypeptide to also retain the N-terminal PH domain of TAPP, particularly for assays or methods performed in cells.

By "able to bind" or "capable of binding" is meant that binding of the TAPP polypeptide to PtdIns(3,4)P₂ (or PTPL1, as appropriate) can be detected using a surface plasmon resonance or (for PtdIns(3,4)P₂) protein lipid overlay technique as described in WO 03/011901. By "substantially unable to bind" is meant that binding of the TAPP polypeptide to the PtdIns(3,4)P₂ or PTPL1 is not detected, or is only weakly detected using a surface plasmon resonance or protein lipid overlay technique as described in WO 03/011901. It is preferred that the TAPP polypeptide binds to PtdIns(3,4)P₂ with at least two, preferably 3, 5, 10, 15, 20, 30 or 50-fold higher affinity than to other phosphoinositides, in particular PtdIns(3,4,5)P₃.

It is preferred that the binding of the TAPP polypeptide to PtdIns(3,4)P₂ has an apparent K_{D} of less than about 2000 nM, 1000 nM or 500 nM, preferably less than about 400 or 350 nM, for example between about 350 nM and 10 nM, when measured using the method described in Example 1 of WO 03/011901. It is preferred that the binding of the said polypeptide to other phosphoinositides, particularly PtdIns(3,4,5)P₃ has an apparent K_{D} of more than about 2000 nM, 1000 nM or 500 nM when measured using the method described in Example 1 of WO 03/011901.

Methods of detecting binding of the TAPP polypeptide (for example a fragment, variant, derivative or fusion of full length TAPP, or fusion of a variant, fragment or derivative) to phospholipids, for example PtdIns(3,4)P₂, are described in Dowler *et al* (*supra*), WO 03/011901 and WO 02/12276 and include a protein-lipid overlay assay in which the lipid is spotted onto a support, for example Hybond-C extra membrane, and protein bound to the support by virtue of interaction with the lipid is detected, for example using an antibody-based method, as well known to those skilled in the art. A surface plasmon resonance assay, for example as described in WO 02/12276 or in Plant et al (1995) Analyt Biochem 226(2), 342-348, may alternatively be used. Methods may make use of a TAPP polypeptide, for example comprising a PH domain, or fragment, variant, derivative or fusion thereof, or fusion of a variant, fragment or derivative that is labelled, for example with a radioactive or fluorescent label. Suitable methods may also be described in, for example, Shirai et al (1998) Biochim Biophys Acta 1402(3), 292-302 (use of an affinity column prepared using phosphatidylinositol analogues) and Rao et al (1999) J Biol Chem 274, 37893-37900 (use of avidin-coated beads bound to biotinylated phosphatidylinositol analogues).

Methods as described in [5] may also be adapted in order to assess the ability of the TAPP polypeptide to bind a phosphoinositide, or to assess the effect of a compound on the ability of the TAPP polypeptide to bind the phosphoinositide, as will be apparent to those skilled in the art.

It is particularly preferred, although not essential, that the variant or fragment or derivative or fusion of the TAPP, or the fusion of the variant or fragment or derivative has at least 30% of the PtdIns(3,4)P₂ binding activity of full-length human TAPP1. It is more preferred if the variant or fragment or derivative or fusion of the said TAPP, or the fusion of the variant or fragment or derivative has at least 50%, preferably at least 70% and more preferably at least 90% of the PtdIns(3,4)P₂ binding activity of full-length human TAPP1.

In addition, as discussed in WO 03/011901, it may be preferred that a variant, fragment, derivative or fusion of TAPP comprises the N-terminal of the two PH domains of TAPP. This PH domain may be capable of interacting with polypeptides, as discussed in WO 02/12276. Alternatively (or in addition), it is preferred that a variant, fragment, derivative or fusion of TAPP comprises one or more proline rich regions found towards the C-terminus of TAPP2 (as discussed in WO 02/12276), which may form a binding site for an SH3 domain.

By the term PTPL1 is included the polypeptides termed PTPL1 or FAP-1 in Saras et al (1994) Cloning and characterization of PTPL1, a protein tyrosine phosphatase with similarities to cytoskeletal-associated proteins. J Biol Chem 269, 24082-9. or Sato et al (1995) FAP-1: a protein tyrosine phosphatase that associates with Fas. Science 268, 411-5. See also accession numbers Q12923, NP_542416, NP_542415, NP_006255, NP_542414, A54971. In relation to polypeptides or structures used in screening methods or assays the term also includes fragments and fusions thereof that comprise the first PDZ domain (and optionally also the fifth PDZ domain), as discussed in Example 1, for example an HA tagged PDZ domain, for example an HA-tagged PDZ domain. The PTPL1 polypeptide preferably also comprises the phosphatase domain, as known to those skilled in the art and as discussed in Example 1. As discussed in, for example, [36], the phosphatase domain is about 250 amino acids in length and has a PTP signature motif V/IHCSAGXGRXG. The cysteine residue is critical for PTP activity. Further examples are considered to include fragments, variants, derivatives or fusions thereof, or fusions of fragments, variants or derivatives, that retain the TAPP-binding properties of full length PTPL1, or the first PDZ domain thereof (ie that are capable of binding to TAPP1 or TAPP2.

Methods for detecting or assessing protein-protein interactions are well known to those skilled in the art, and include techniques described in Example 1. Suitable methods include yeast two-hybrid interactions, co-purification, ELISA, co-immunoprecipitation methods and cellular response assays. Cellular response assays may be carried out in a variety of cell types, for example in adipocytes or adipocyte cell lines, in a skeletal muscle cell line (such as the L6 myotubule cell line), liver cells or liver cell lines or cancer cells or cancer cell lines. Examples of suitable cell types are used in Example 1.

Other methods of detecting polypeptide/polypeptide interactions include ultrafiltration with ion spray mass spectroscopy/HPLC methods or other physical and analytical methods. Fluorescence Energy Resonance Transfer (FRET) methods, for example, well known to those skilled in the art, may be used, in which binding of two fluorescent labelled entities may be measured by measuring the interaction of the fluorescent labels when in close proximity to each other.

This may be done in a whole cell system or using purified or partially purified components. Similarly, expression of a protein encoded by an RNA transcribed from a promoter regulated by TAPP/PTPL1 may be measured. The protein may be one that is physiologically regulated by the polypeptide or may be a "reporter" protein, as well known to those skilled in the art (ie a recombinant construct may be used). A reporter protein may be one whose activity may easily be assayed, for example (β-galactosidase, chloramphenicol acetyltransferase or luciferase (see, for example, Tan *et al*(1996)). In a further example, the reporter gene may be fatal to the cells, or alternatively may allow cells to survive under otherwise fatal conditions. Cell survival can then be measured, for example using colorimetric assays for mitochondrial activity, such as reduction of WST-1 (Boehringer). WST-1 is a formosan dye that undergoes a change in absorbance on receiving electrons via succinate dehydrogenase.

Promoters whose activity may be regulated by a signalling pathway in which TAPP/PTPL1 may be involved may be identified using microarray technology, as known to those skilled in the art, in which the expression of multiple genes may be examined simultaneously, for example in stimulated and unstimulated cells expressing the wild-type polypeptide or a dominant negative mutation. An example of a suitable dominant negative mutant of TAPP is a fragment of TAPP lacking the C-terminal PtdIns(3,4)P₂-binding PH domain, or a TAPP polypeptide in which the C-terminal PtdIns(3,4)P₂-binding domain is mutated or otherwise disrupted so that it does not bind PtdIns(3,4)P₂, for example as described in WO02/12276. For example, mutation of the conserved Arg212 to Leu (or equivalent residue in TAPP2) in the Putative PtdIns(3,4,5)P₃ Binding Motif (PPBM) of the C-terminal PH domain of TAPP1 abolished the interaction of both full length TAPP1 and the isolated C-terminal PH domain with PtdIns(3,4)P₂. The mutation to Leu of the conserved Trp residue (Trp281) found in all PH domains, also abolished the interaction of the isolated C-terminal PH domain of TAPP1 with PtdIns(3,4)P₂. Differences in expression patterns between the different cells/activation states indicate genes/promoters which TAPP/PTPL1 may regulate. Thus, transcription of these genes may be assessed or the promoter for such a gene may be used in a reporter construct as described above.

Insulin exerts important effects on gene expression in multiple tissues (O'Brien, R. M. & Granner, D. K (1996) Physiol. Rev. 76, 1109-1161, and as discussed above). In the liver, insulin suppresses the expression of a number of genes which contain a conserved insulin response sequence (IRS)¹ (CAAAAC/TAA), including insulin-like growth factor binding protein-1 (IGFBP-1), apolipoprotein CIII (apoCIII), phosphoenol-pyruvate carboxykinase (PEPCK) and glucose-6 phosphatase (G6Pase) (Goswami, R et al (1994) Endocrinol. 134, 2531-2539; Suwanickul, A et al (1993) J. Biol Chem. 268, 17063-17068; Li, W. W et al (1995) J. Clin. Invest 96, 2601-2605; O'Brien, R. M et al (1990) Science 249, 533-537; Streeper, R. S et al (1997) J. Biol Chem. 272, 11698-11701). Thus, transcription of these genes may be assessed or promoters from these genes may be used in a reporter construct as described above. Microarray technology may be used in assessing transcription of genes or reporter constructs, as known to those skilled in the art.

The transcription of a gene indicated above (or any other that is regulated by cellular stress, a growth factor or insulin signalling) may be measured by measurement of changes in the enzymatic or other activity of the said gene product, for example in a cell. Suitable methods will be well known to those skilled in the art. Measurements may be made of p70S6 kinase or PKB kinase activity, or of cell survival, glucose uptake, glycogen sythesis or protein synthesis.

It will be necessary to perform various control assays, as known to those skilled in the art, in order to determine that a compound is affecting signalling via TAPP/PTPL1, rather than having some other effect on processes leading to whatever measurement is made. For example, it may be necessary to determine what effect the compound being tested has on the activity rather than the activation of a polypeptide, for example a protein kinase, that may be acting downstream (in the signalling pathway) of TAPP/PTPL1 but upstream of the effect being measured.

It will be understood that the method is for identifying compounds for modulating the cellular activity or location of PTPL1. It is desirable that the compounds are able to modulate the cellular activity or location of endogenous PTPL1. Thus it will be understood that reagents (including any fragment, derivative, variant or fusion of the polypeptides or fusion of a variant, fragment or derivative) and conditions used in screening methods as discussed further below may be chosen such that the interactions between the said polypeptides, or between TAPP and PtdIns(3,4)P₂, or interactions with other cellular components are substantially the same as between the wild-type, preferably human polypeptides (for example PTPL1, TAPP1 or TAPP2) and the phosphoinositide or interacting components *in vivo*. Thus, it may be desirable to conduct screens using full length wild-type polypeptides in cells; or to check at least some of the results of screens in such a system.

The sequences of human and mouse TAPP1 and TAPP2 are shown in Figures 5 and 6 of WO 03/011901.

By "variants" of a polypeptide we include insertions, deletions and substitutions, either conservative or non-conservative. In particular we include variants of the polypeptide where such changes do not substantially alter the activity of TAPP or PTPL1, as described above.

By "conservative substitutions" is intended combinations such as Gly, Ala; Val, Ile, Leu; Asp, Glu; Asn, Gln; Ser, Thr; Lys, Arg; and Phe, Tyr.

It is particularly preferred if the PTPL1 or TAPP variant has an amino acid sequence which has at least 65% identity with the amino acid sequence of (as appropriate) PTPL1 or TAPP1 or TAPP2 referred to above, more preferably at least 70%, 71%, 72%, 73% or 74%, still more preferably at least 75%, yet still more preferably at least 80%, in further preference at least 85%, in still further preference at least 90% and most preferably at least 95% or 97% identity with the amino acid sequence defined above.

The sequence of human PTPL1 is discussed in references 28, 29 and 30 (Sato et al (1995) FAP-1: a protein tyrosine phosphatase that associates with Fas. Science 268, 411-5; Yang et al (1991) Isolation of a cDNA clone encoding a human protein-tyrosine phosphatase with homology to the cytoskeletal-associated proteins band 4.1, ezrin, and talin. Proc Natl Acad Sci U S A 88, 5949-53; Gu et al (1991) Identification, cloning, and expression of a cytosolic megakaryocyte protein-tyrosine-phosphatase with sequence homology to cytoskeletal protein 4.1. Proc Natl Acad Sci U S A 88, 5867-71).

It is still further preferred if the TAPP variant has an amino acid sequence which has at least 65% identity with the amino acid sequence of the C-terminal PH domain, particularly the residues forming the phosphoinositide binding site, of TAPP in the appropriate sequence referred to above, more preferably at least 70%, 71%, 72%, 73% or 74%, still more preferably at least 75%, yet still more preferably at least 80%, in further preference at least 83 or 85%, in still further preference at least 90% and most preferably at least 95% or 97% identity with the amino acid sequence defined above. It will be appreciated that the PH domain may be readily identified by a person skilled in the art, for example using sequence comparisons, for example as described in Dowler *et al* (2000) *supra* and WO 02/12276.

It is still further preferred if the PTPL1 variant has an amino acid sequence which has at least 65% identity with the amino acid sequence of the first PDZ domain (and optionally also the fifth PDZ domain) of PTPL1 in the appropriate sequence referred to above, more preferably at least 70%, 71%, 72%, 73% or 74%, still more preferably at least 75%, yet still more preferably at least 80%, in further preference at least 83 or 85%, in still further preference at least 90% and most preferably at least 95% or 97% identity with the amino acid sequence defined above. It will be appreciated that the PDZ domains may be readily identified by a person skilled in the art, for example using sequence comparisons, for example as discussed in Example 1.

The percent sequence identity between two polypeptides may be determined using suitable computer programs, for example the GAP program of the University of Wisconsin Genetic Computing Group and it will be appreciated that percent identity is calculated in relation to polypeptides whose sequence has been aligned optimally.

The alignment may alternatively be carried out using the Clustal W program (Thompson et al (1994) Nucl Acid Res 22, 4673-4680). The parameters used may be as follows:
Fast pairwise alignment parameters: K-tuple(word) size; 1, window size; 5, gap penalty; 3, number of top diagonals; 5. Scoring method: x percent.
Multiple alignment parameters: gap open penalty; 10, gap extension penalty; 0.05.
Scoring matrix: BLOSUM.

It is preferred that the PTPL1 or TAPP is a polypeptide which consists of the amino acid sequence of the PTPL1 or TAPP1 or TAPP2 (preferably TAPP1) sequence referred to above or naturally occurring allelic variants thereof. It is preferred that the naturally occurring allelic variants are mammalian, preferably human, but may alternatively be homologues from other organisms.

Recombinant polypeptides may be used, as well known to those skilled in the art. The TAPP or PTPL1 may be FLAG-tagged, Myc epitope-tagged or His-tagged or GST-tagged, for example as described in Example 1 or in WO 03/011901 or WO 02/12276. It may be a GFP (green fluorescent protein) fusion, as known to those skilled in the art.

In an embodiment the method of the first aspect of the invention may further comprise the step (as discussed in WO 03/011901) of comparing a three-dimensional structure of at least a part of a PtdIns (3,4,5)P₃ binding domain with a three-dimensional structure of a compound, and a compound that is predicted to interact with the said phosphoinositide binding domain (or site or part thereof) of TAPP with a higher affinity than it is predicted to interact with the said PtdIns (3,4,5)P₃ binding domain is selected.

Preferably the three-dimensional structure of at least a part of the PtdIns (3,4,5)P₃ binding domain is a three-dimensional structure of at least a part of the PtdIns (3,4,5)P₃ binding site of the PtdIns (3,4,5)P₃ binding domain and a compound that is predicted to interact with the said phosphoinositide binding domain (or site or part thereof) of TAPP with a higher affinity than it is predicted to interact with the said PtdIns (3,4,5)P₃ binding site is selected. Still more preferably, the three-dimensional structure of at least a part of the PtdIns (3,4,5)P₃ binding site is a three-dimensional structure of the part of the PtdIns (3,4,5)P₃ binding site that is defined by loops β1-β2, β-3-β4, β6-β7 of a PtdIns (3,4,5)P₃ binding domain and a compound that is predicted to interact with the said phosphoinositide binding domain (or site or part thereof) of TAPP with a higher affinity than it is predicted to interact with the said part of the PtdIns (3,4,5)P₃ binding site is selected.

Thus, it is preferred that the structures of the portion of the TAPP phosphoinositide binding site that is responsible for its specificity for PtdIns(3,4)P₂, and the portion of the PtdIns(3,4,5)P₃ binding site that is responsible for its specificity for PtdIns(3,4,5)P₃, are compared with the structure of the test compound. A compound is selected that matches this portion of the PtdIns(3,4)P₂ binding site more closely than it matches this portion of the PtdIns(3,4,5)P₃ binding site. Preferences for the PtdIns (3,4,5)P₃ binding domain are as indicated in WO 03//011901.

The three-dimensional structures that are compared may be predicted three-dimensional structures or may be three-dimensional structures that have been determined, for example by techniques such as X-ray crystallography, as well known to those sldlled in the art and as described in WO 03/011901. The three-dimensional structures may be displayed by a computer in a two-dimensional form, for example on a computer screen. The comparison may be performed using such two-dimensional displays.

It is preferred that the structure is of the PtdIns(3,4)P₂ binding domain of TAPP, still more preferably TAPP1. Most preferably, it is the structure reported in Example 1 and Figure 9 of WO 03/011901. Alternatively, the structure may be a structure of another PtdIns(3,4)P₂-specific binding domain obtained or obtainable by modelling based on the structure of the PtdIns(3,4)P₂ binding domain of TAPP and any other relevant structural information, as discussed in WO 03/011901

In an embodiment the method of the first aspect of the invention may comprise the step of determining whether a test compound modulates the interaction of PTPL1 with TAPP; or modulates or mimics the interaction of TAPP with PtdIns(3,4)P₂; or modulates the cellular location of TAPP.

Methods for assessing the PTPL1-TAPP interaction and the effect of a test compound on such interaction will be apparent to those skilled in the art and include methods for assessing protein-protein interactions as described herein. Methods for assessing the TAPP-PtdIns(3,4)P₂ interaction and the effect of a test compound on such interaction will also be apparent to those skilled in the art and include methods for assessing protein-phosphoinositide interactions as described herein. Methods for assessing cellular location, for example of TAPP, will also be apparent to those skilled in the art and include methods such as those described in Kimber *et al* (2002) [17], for example methods in which a fluorescent-tagged TAPP polypeptide, for example a GFP (or YFP) tagged TAPP polypeptide is used.

A further aspect of the invention provides a method for selecting a compound for modulating signalling via PtdIns(3,4)P₂, the method comprising the step of identifying a compound which modulates the interaction between TAPP and PTPL1 Methods for assessing the effect of a compound on the interaction between TAPP and PTPL1 will be apparent to those skilled in the art, as discussed above. For example, co-immunoprecipitation, biaCore or calorimetric analysis may be used. Computer modelling techniques may also be useful, as will be apparent to those skilled in the art.

As discussed in WO 03/011901, a polycarboxylate molecule is able to bind to a PH domain, at least with low affinity. By comparing the structure of a compound with the structure of a polycarboxylate, preferably citrate, still more preferably in the conformation found in the complex with the PH domain, as described in WO 03/011901, it may be possible to determine whether and with what affinity the compound is likely to interact with the PH domain and therefore whether it may be useful in modulating (via TAPP) the activity of PTPL1.

A polypeptide comprising an amino acid sequence (preferably C-terminal amino acid sequence) corresponding to the consensus sequence Ser/Thr-Xaa-Val/Ile, for example SDV, or a corresponding peptidomimetic compound, may be capable of inhibiting binding of TAPP, for example TAPP1 or TAPP2, to PTPL1. Such a polypeptide or peptidomimetic compound may be useful in itself or may be useful in selecting or designing a more useful compound.

By "design" we include the meaning that the agent is made or modified so that it contains one or more functional groups which it is expected or known will bind all or part of the desired phosphoinositide binding domain, particularly phosphoinositide binding site, still more particularly part of a phosphoinositide binding site that is important in determining what type(s) of phosphoinositide are bound.

Skilled persons will be aware of a range of methods which can be used in methods of the present invention to design or select agents based on the knowledge of the PtdIns(3,4)P₂ binding site and the interaction with a polycarboxylate. Such methods, including screening methods and computer aided design methods, are discussed in, for example, WO 03/011901.

By "selection" we include affinity purification techniques for identifying agents which are capable of binding to all or part of, for example, the PtdIns(3,4)P₂ binding site. Examples include affinity chromatography and phage display technologies.

The agents identified or obtained according to the above aspects of the invention (as well as the screening methods provided by further aspects of the invention, as discussed below) may be a drug-like compound or lead compound for the development of a drug-like compound. Thus, the methods may be methods for identifying a drug-like compound or lead compound for the development of a drug-like compound.

The term "drug-like compound" is well known to those skilled in the art, and may include the meaning of a compound that has characteristics that may make it suitable for use in medicine, for example as the active ingredient in a medicament Thus, for example, a drug-like compound may be a molecule that may be synthesised by the techniques of organic chemistry, less preferably by techniques of molecular biology or biochemistry, and is preferably a small molecule, which may be of less than 5000 daltons and which may be water-soluble. A drug-like compound may additionally exhibit features of selective interaction with a particular protein or proteins and be bioavailable and/or able to penetrate target cellular membranes, but it will be appreciated that these features are not essential.

The term "lead compound" is similarly well know to those skilled in the art, and may include the meaning that the compound, whilst not itself suitable for use as a drug (for example because it is only weakly potent against its intended target, non-selective in its action, unstable, poorly soluble, difficult to synthesise, too toxic or has poor bioavailability) may provide a starting-point for the design of other compounds that may have more desirable characteristics.

The compounds identified in the methods of the invention may themselves be useful as a drug or they may represent lead compounds for the design and synthesis of more efficacious compounds.

The compound may be an antibody that binds to the phosphoinositide binding site of TAPP. Such an antibody may inhibit the binding of PtdIns(3,4)P₂ to TAPP, or may mimic the effect of binding of PtdIns(3,4)P₂ to TAPP. The compound may be an antibody that binds to the first (or fifth) PDZ domain of PTPL1 or to the C-terninal portion of TAPP, thereby inhibiting the interaction of TAPP with PTPL1.

Antibodies reactive towards the said polypeptides may be made by methods well known in the art. In particular, the antibodies may be polyclonal or monoclonal.

Suitable monoclonal antibodies which are reactive towards the said polypeptide may be prepared by known techniques, for example those disclosed in "Monoclonal Antibodies: A manual of techniques", H Zola (CRC Press, 1988) and in "Monoclonal Hybridoma Antibodies: Techniques and Applications", SGR Hurrell (CRC Press, 1982).

Techniques for preparing antibodies are well known to those skilled in the art, for example as described in Harlow, ED & Lane, D "Antibodies: a laboratory manual" (1988) New York Cold Spring Harbor Laboratory.

By the term "antibody" is included antibody-like molecules, including, for example, antibody fragments or derivatives which retain their antigen-binding sites, synthetic antibody-like molecules such as single-chain Fv fragments (ScFv) and domain antibodies (dAbs), and other molecules with antibody-like antigen binding motifs.

The selected or designed compound may be synthesised (if not already synthesised) and tested for its effect on TAPP or PTPL1, for example its effect on cellular location of PTPL1. The compound may be tested in a screening method of the invention.

The method may make use of a method comprising the step of determining the effect of the compound on the activity of, or ability of the compound to bind to, (1) a mutated TAPP or mutated phosphoinositide binding domain of TAPP, mutated at a residue of loop β1-β2, β-3-β4 or β6-β7 defining at least part of the phosphoinositide binding site of TAPP, and optionally also (2) TAPP or the phosphoinositide binding domain of TAPP which is not mutated at a said residue, and selecting a compound which affects to different extents the activity of, or binds with different affinities to, the unmutated TAPP/domain and the mutated TAPP/domain.

Preferably the effect of the compound on the phosphoinositide binding activity of the mutated TAPP/domain and optionally unmutated TAPP/domain is determined. Alternatively, the effect of the compound on an activity that is modulated by phosphoinositide binding to TAPP may be measured. Examples of such activities are noted above and may include changes in cellular location.

Depending upon the parameters measured, the screening method may identify compounds which inhibit binding of phosphoinositides to TAPP, and compounds which mimic binding of phosphoinositides to TAPP, as will be apparent to those skilled in the art. It will be appreciated that screens in which binding of compounds to TAPP is measured may be used in conjunction with screens in which the effect of compounds (for example compounds identified in the binding screen(s)) on one or more activities of TAPP (for example phosphoinositide binding activity or cellular location of TAPP or PTPL1) is measured.

By "mimics the effect" of the interaction of a phosphoinositide (for example PtdIns(3,4)P₂) with the TAPP is meant that the compound has a quantitative or qualitative effect on the TAPP, for example on its cellular location, that is the same as an effect of the phosphoinositide on the TAPP, for example on its cellular location.

It is preferred that the mutated TAPP/domain is capable of binding to PtdIns(3,4,5)P₃. Examples of such mutated TAPP are discussed in Example 1 of WO 03/011901. It is particularly preferred that the TAPP/domain is mutated at a residue of loop β1-β2, for example at the residue equivalent to Arg212, Ala203 and/or Val204 of full length TAPP1. It is most preferred that the TAPP is mutated at the residue equivalent to Ala203 of full length TAPP1, or at the residues equivalent to Ala203 and Val204 of full length TAPP1, as discussed in Example 1 of WO 03/011901.

Any convenient method may be used to measure the interaction of the TAPP domain with the compound or the effect of such binding. Conveniently, the appropriate methods make use of the methods described in, for example, WO 03/011901, for detecting and/or quantifying the interaction between a polypeptide and a phospholipid, for example a protein-lipid overlay or surface plasmon resonance method, as discussed above. It is preferred that a GST-tagged fusion polypeptide is used. Methods in which radioactively or fluorescently labelled lipids are used may also be useful.

Methods of detecting protein-protein interactions are well known to those skilled in the art. Suitable methods include yeast two-hybrid interactions, co-purification, ELISA, co-immunoprecipitation methods and cellular response assays. Cellular response assays may be carried out in a variety of cell types, for example insulin-responsive cells. For example, assays may be carried out in adipocytes or adipocyte cell lines, in a skeletal muscle cell line (such as the L6 myotubule cell line), liver cells or liver cell lines or cancer cells or cancer cell lines.

Methods involving BiaCore or FRET measurements may be particularly useful for assessing molecular interactions or cellular location.

Platelets may be preferred when the polypeptide is TAPP. NIH Swiss mouse embryo cells NIH/3T3 (available from the American Type Culture Collection (ATCC) of Rockville, MD, USA (ATCC) as CRL 1658) and human embryonic kidney 293 cells (also available from the ATCC) are examples of cell lines that may be used when investigating the effect of hydrogen peroxide or other cellular stress treatment (which may lead to production of PtdIns(3,4)P₂, as noted above).

The screening methods of the may be performed *in vitro*, either in intact cells or tissues, with broken cell or tissue preparations or at least partially purified components. The cells, tissues or organisms in/on which the method is performed are preferably transgenic. In particular they may be transgenic for TAPP and/or PTPL1, for example may express a mutated or truncated TAPP or PTPL1 in place of, or in addition to, endogenous TAPP or PTPL1. They may express human TAPP or PTPL1 (or, for example, a mutant or fragment thereof) in place of endogenous TAPP or PTPL1. Levels of endogenous TAPP or PTPL1 may be reduced by gene knock-out or by techniques such as RNAi-mediated knock-down, for example as described in Example 1.

In a preferred embodiment, the interaction of the TAPP or PTPL1 with the compound is measured using fluorescence resonance energy transfer (FRET). Alternatively, the interaction of the TAPP or PTPL1 with the compound is measured using surface plasmon resonance. The interaction of the compound with TAPP or PTPL1 may be measured by assessing the effect of the compound on the interaction of TAPP with PTPL1 and/or with a phosphoinositide.

Techniques described in WO 02/12276, adapted in view of the present teaching, may also be useful.

It will be appreciated that screening assays which are capable of high throughput operation will be particularly preferred. Examples may include cell based assays and protein-protein binding assays. An SPA-based (Scintillation Proximity Assay; Amersham International) system may be used, as well know to those skilled in the art. For example, either of the polypeptides or a phosphoinositide may be immobilised on the SPA beads and the ability of the test compound to disrupt the interaction between the polypeptide and phosphoinositide (or between the polypeptides) may be measured. Phosphoinositides or analogues thereof may be immobilised on SPA beads, for example using methods as described in Shirai et al (1998) Biochim Biophys Acta 1402(3), 292-302 or in Rao et al (1999) J Biol Chem 274, 37893-37900.

The ability of the compound to bind to the said polypeptide may be measured by measuring the ability of the compound to disrupt or prevent the interaction between the polypeptide and the interacting phosphoinositide, or between the polypeptides.

The binding constant for the binding of the compound to the relevant polypeptide may be determined. Suitable methods for detecting and/or measuring (quantifying) the binding of a compound to a polypeptide are well known to those skilled in the art and may be performed, for example using a method capable of high throughput operation, for example a chip-based method in which the compounds to be tested are immobilised in a microarray on a solid support, as known to those skilled in the art.

The following techniques may be useful in assessing compound or phosphoinositide binding: calorimetric measurements, measurements using tritiated or other labelled phosphoinositides or headgroups, gel filtration methods (to separate bound and unbound compound or phosphoinositide), protein- lipid overlay (for example in a 384-well assay format).

It will be appreciated that the screening assays of the invention are useful for identifying compounds which may be useful in the treatment of diabetes (for example Type II diabetes, but also Type I diabetes), defects of glycogen metabolism, cancer (for example hormone dependent cancers), inflammatory conditions, ischaemic conditions, for example stroke, thrombosis or tendency to thrombosis (for example useful as an antithrombotic agent).

It is considered that compounds which disrupt the interaction of TIPP with PTPL1 may inhibit the trans location of PTPL1 to the plasma membrane , thereby slowing the phosphatase-catalysed "switching off' of PI 3-kinase-dependent or other signalling pathways by dephosphorylation of receptor tyrosine kinases or tyrosine phosphorylated adaptor proteins such as IRS1 or IRS2. Slowing such dephosphorylation is considered to prolong the signalling and therefore have the effect of amplifying or prolonging the effect of the signal initiator on the cell, for example increasing the effect of insulin (or a growth factor such as epidermal growth factor (EGF) or insulin-like growth factor (IGF)) on a cell responsive thereto. Accordingly, a compound that disrupts the interaction of TPP with PTPL1 is considered to be useful in treating diabetes (principally Type II but also Type I), inhibition of apoptosis, for example in the treatment of ischaemic disease, wound healing or nerve regeneration.

Compounds which promote the interaction of TAPP with PTPL1 may in contrast promote the translocation of PTPL1 to the plasma membrane, and may therefore inhibit or shorten the effect of the signal initiator on the cell, for example decreasing the effect of insulin (or a growth factor such as EGF or IGF) on a cell responsive thereto. Such a compound is considered to be useful in promoting apoptosis, for example in treating cancer or in the resolution of inflammation.

Promotion of TAPP activity/interaction with PTPL1 may inhibit platelet activation, which may be useful in reducing or preventing thrombosis. This may be important in patients at risk of thrombosis (for example obese patients or those with a history of thrombosis) and/or before, during or after surgery.

Thus, the "drug-like compounds" and "lead compounds" identified in the screening assays of the invention are suitably screened in further screens to determine their potential usefulness in treating diabetes, defects of glycogen metabolism, cancer, inflammatory conditions, ischaemic conditions, for example stroke, or thrombosis or tendency to thrombosis. Additional screens which may be carried out include determining the effect of the compounds on blood glucose levels, tumour growth or blood clotting tendency/time, as appropriate. This may typically be done in rodents.

Over-expression of a substantially full-length native TAPP polypeptide may be useful in inhibiting or shortening the effect of the signal initiator on the cell, for example decreasing the effect of insulin (or a growth factor such as EGF) on a cell responsive thereto. This is considered to be useful in promoting apoptosis, for example in treating cancer or in the resolution of inflammation. A still further aspect of the invention provides the use of a compound capable of promoting the expression of TAPP in the manufacture of a medicament for promoting apoptosis, for example in treating cancer or in the resolution of inflammation.

Expression of a truncated or mutant TAPP molecule that is capable of interacting with PTPL1 (for example retains the C-terminal amino acids required for interaction with the PTPL1 PDZ domain(s)) but not of binding to PtdIns(3,4)P₂ (for example due to lacking the C-terminal PH domain, or having a mutated or disrupted PH domain that does not bind to PtdIns(3,4)P₂, as discussed above) may be useful in inhibiting the translocation of PTPL1 to the plasma membrane, thereby slowing the phosphatase-catalysed "switching off" of PI 3-kinase-dependent or other signalling pathways by dephosphorylation of receptor tyrosine kinases or tyrosine phosphorylated adaptor proteins such as IRS1 or IRS2. Accordingly, expression of such a TAPP polypeptide is considered to be useful in treating diabetes (principally Type II but also Type I), or in inhibition of apoptosis, for example in the treatment of ischaemic disease, wound healing or nerve regeneration. Reducing apoptosis may be useful following ischaemic injury, for example stroke or myocardial infarction, and in tissue repair. It may also be useful in the treatment of patient before, after or during heart surgery. A still further aspect of the invention provides the use of a compound capable of promoting the expression of a truncated or mutant TAPP polypeptide that is capable of interacting with PTPL1 but not of binding to PtdIns(3,4)P₂ (for example a gene therapy vector capable of expressing such a truncated or mutant TAPP polypeptide) in the manufacture of a medicament for the treatment of diabetes (principally Type II but also Type I), or for inhibition of apoptosis, for example in the treatment of ischaemic disease, wound healing or nerve regeneration.

Disruption of expression of TAPP may similarly be useful in treating diabetes (principally Type II but also Type I), or in inhibition of apoptosis, for example in the treatment of ischaemic disease, wound healing or nerve regeneration. Expression may be disrupted by a compound such as an RNAi molecule, for example as described in Example 1, or may be an antisense molecule or ribozyme directed (for example, capable of binding to a polynucleotide encoding TAPP under physiological conditions) against a polynucleotide encoding TAPP (for example TAPP1 or TAPP2). A still further aspect of the invention provides the use of a compound (for example a nucleic acid molecule) capable of disrupting the expression of TAPP in the manufacture of a medicament for the treatment of diabetes (principally Type II but also Type I), or for inhibition of apoptosis, for example in the treatment of ischaemic disease, wound healing or nerve regeneration.

It will be appreciated that the nucleic acid may be an antisense oligonucleotide, for example an antisense oligonucleotide directed against a TAPP gene, for example the TAPP1 or TAPP2 gene. Antisense oligonucleotides are single-stranded nucleic acid, which can specifically bind to a complementary nucleic acid sequence. By binding to the appropriate target sequence, an RNA-RNA, a DNA-DNA, or RNA-DNA duplex is formed. These nucleic acids are often termed "antisense" because they are complementary to the sense or coding strand of the gene. Recently, formation of a triple helix has proven possible where the oligonucleotide is bound to a DNA duplex. It was found that oligonucleotides could recognise sequences in the major groove of the DNA double helix. A triple helix was formed thereby. This suggests that it is possible to synthesise a sequence-specific molecules which specifically bind double-stranded DNA *via* recognition of major groove hydrogen binding sites.

By binding to the target nucleic acid, the above oligonucleotides can inhibit the function of the target nucleic acid. This could, for example, be a result of blocking the transcription, processing, poly(A)addition, replication, translation, or promoting inhibitory mechanisms of the cells, such as promoting RNA degradations.

Antisense oligonucleotides are prepared in the laboratory and then introduced into cells, for example by microinjection or uptake from the cell culture medium into the cells, or they are expressed in cells after transfection with plasmids or retroviruses or other vectors carrying an antisense gene. Antisense oligonucleotides were first discovered to inhibit viral replication or expression in cell culture for Rous sarcoma virus, vesicular stomatitis virus, herpes simplex virus type 1, simian virus and influenza virus. Since then, inhibition of mRNA translation by antisense oligonucleotides has been studied extensively in cell-free systems including rabbit reticulocyte lysates and wheat germ extracts. Inhibition of viral function by antisense oligonucleotides has been demonstrated *in vitro* using oligonucleotides which were complementary to the AIDS HIV retrovirus RNA (Goodchild, J. 1988 "Inhibition of Human Immunodeficiency Virus Replication by Antisense Oligodeoxynucleotides", Proc. Natl. Acad. Sci. (USA) 85(15), 5507-11). The Goodchild study showed that oligonucleotides that were most effective were complementary to the poly(A) signal; also effective were those targeted at the 5' end of the RNA, particularly the cap and 5' untranslated region, next to the primer binding site and at the primer binding site. The cap, 5' untranslated region, and poly(A) signal lie within the sequence repeated at the ends of retrovirus RNA (R region) and the oligonucleotides complementary to these may bind twice to the RNA.

Oligonucleotides are subject to being degraded or inactivated by cellular endogenous nucleases. To counter this problem, it is possible to use modified oligonucleotides, eg having altered internucleotide linkages, in which the naturally occurring phosphodiester linkages have been replaced with another linkage. For example, Agrawal et al (1988) Proc. Natl. Acad. Sci. USA 85, 7079-7083 showed increased inhibition in tissue culture of HIV-1 using oligonucleotide phosphoraraidates and phosphorothioates. Sarin et al (1988) Proc. Natl. Acad Sci. USA 85, 7448-7451 demonstrated increased inhibition of HIV-1 using oligonucleotide methylphosphonates. Agrawal et al (1989) Proc. Natl. Acad. Sci. USA 86, 7790-7794 showed inhibition of HIV-1 replication in both early-infected and chronically infected cell cultures, using nucleotide sequence-specific oligonucleotide phosphorothioates. Leither et al (1990) Proc. Natl. Acad. Sci. USA 87, 3430-3434 report inhibition in tissue culture of influenza virus replication by oligonucleotide phosphorothioates.

Oligonucleotides having artificial linkages have been shown to be resistant to degradation *in vivo.* For example, Shaw et al (1991) in Nucleic Acids Res. 19, 747-750, report that otherwise unmodified oligonucleotides become more resistant to nucleases *in vivo* when they are blocked at the 3□ end by certain capping structures and that uncapped oligonucleotide phosphorothioates are not degraded *in vivo.*

A detailed description of the H-phosphonate approach to synthesising oligonucleoside phosphorothioates is provided in Agrawal and Tang (1990) Tetrahedron Letters 31, 7541-7544. Syntheses of oligonucleoside methylphosphonates, phosphorodithioates, phosphoramidates, phosphate esters, bridged phosphoramidates and bridge phosphorothioates are known in the art. See, for example, Agrawal and Goodchild (1987) Tetrahedron Letters 28, 3539; Nielsen et al (1988) Tetrahedron Letters 29, 2911; Jager et al (1988) Biochemistry 27, 7237; Uznanski et al (1987) Tetrahedron Letters 28, 3401; Bannwarth (1988) Helv. Chim. Acta. 71, 1517; Crosstick and Vyle (1989) Tetrahedron Letters 30, 4693; Agrawal et al (1990) Proc. Natl. Acad. Sci. USA 87 1401-1405. Other methods for synthesis or production also are possible. In a preferred embodiment the oligonucleotide is a deoxyribonucleic acid (DNA), although ribonucleic acid (RNA) sequences may also be synthesised and applied.

The oligonucleotides useful in the invention preferably are designed to resist degradation by endogenous nucleolytic enzymes. *In vivo* degradation of oligonucleotides produces oligonucleotide breakdown products of reduced length. Such breakdown products are more likely to engage in non-specific hybridization and are less likely to be effective, relative to their full-length counterparts. Thus, it is desirable to use oligonucleotides that are resistant to degradation in the body and which are able to reach the targeted cells. The present oligonucleotides can be rendered more resistant to degradation *in vivo* by substituting one or more internal artificial internucleotide linkages for the native phosphodiester linkages, for example, by replacing phosphate with sulphur in the linkage. Examples of linkages that may be used include phosphorothioates, methylphosphonates, sulphone, sulphate, ketyl, phosphorodithioates, various phosphoramidates, phosphate esters, bridged phosphorothioates and bridged phosphoramidates. Such examples are illustrative, rather than limiting, since other internucleotide linkages are known in the art. See, for example, Cohen, (1990) Trends in Biotechnology*.* The synthesis of oligonucleotides having one or more of these linkages substituted for the phosphodiester internucleotide linkages is well known in the art, including synthetic pathways for producing oligonucleotides having mixed internucleotide linkages.

Oligonucleotides can be made resistant to extension by endogenous enzymes by. "capping" or incorporating similar groups on the 5' or 3' terminal nucleotides. A reagent for capping is commercially available as Amino-Link II^{™} from Applied BioSystems Inc, Foster City, CA. Methods for capping are described, for example, by Shaw et al (1991) Nucleic Acids Res. 19, 747-750 and Agrawal et al (1991) Proc. Natl. Acad. Sci. USA 88(17), 7595-7599 .

A further method of making oligonucleotides resistant to nuclease attack is for them to be "self-stabilised" as described by Tang et al (1993) Nucl. Acids Res. 21, 2729-2735 . Self-stabilised oligonucleotides have hairpin loop structures at their 3' ends, and show increased resistance to degradation by snake venom phosphodiesterase, DNA polymerase I and fetal bovine serum. The self-stabilised region of the oligonucleotide does not interfere in hybridization with complementary nucleic acids, and pharmacokinetic and stability studies in mice have shown increased *in vivo* persistence of self-stabilised oligonucleotides with respect to their linear counterparts.

It will be appreciated that antisense agents also include larger molecules which bind to the TAPP polypeptide mRNA or genes and substantially prevent expression of said TAPP polypeptide mRNA or genes and substantially prevent expression of said TAPP polypeptide. Thus, expression of an antisense molecule which is substantially complementary to said TAPP polypeptide is envisaged as part of the invention.

The said larger molecules (or molecules encoding TAPP polypeptides, as indicated above, for example full length native TAPP, or a truncated or mutant TAPP molecule that is capable of interacting with PTPL1 but not of binding to PtdIns(3,4)P₂, as discussed above) may be expressed from any suitable genetic construct as is described below and delivered to the patient Typically, the genetic construct which expresses the antisense molecule comprises at least a portion of the said interacting polypeptide coding sequence operatively linked to a promoter which can express the antisense molecule in the cell. Suitable promoters will be known to those skilled in the art, and may include promoters for ubiquitously expressed, for example housekeeping genes or for tissue-specific genes, depending upon where it is desired to express the antisense (or other) molecule.

Although the genetic construct can be DNA or RNA it is preferred if it is DNA.

Preferably, the genetic construct is adapted for delivery to a human cell.

Means and methods of introducing a genetic construct into a cell in an animal body are known in the art. For example, the constructs of the invention may be introduced into the cells by any convenient method, for example methods involving retroviruses, so that the construct is inserted into the genome of the (dividing) cell.

Other methods involve simple delivery of the construct into the cell for expression therein either for a limited time or, following integration into the gnome, for a longer time. An example of the latter approach includes liposomes (Nassander et al (1992) Cancer Res. 52, 646-653). Other methods of delivery include adenoviruses carrying external DNA via an antibody-polylysine bridge (see Curiel Prog. Med. Virol. 40, 1-18) and transferrin-polycation conjugates as carriers (Wagner et al (1990) Proc. Natl. Acad. Sci. USA 87, 3410-3414). The DNA may also be delivered by adenovirus wherein it is present within the adenovirus particle. It will be appreciated that "naked DNA" and DNA complexed with cationic and neutral lipids may also be useful in introducing the DNA of the invention into cells of the patient to be treated. Non-viral approaches to gene therapy are described in Ledley (1995) Human Gene Therapy 6, 1129-1144. Alternative targeted delivery systems are also known such as the modified adenovirus system described in WO 94/10323 wherein, typically, the DNA is carried within the adenovirus, or adenovirus-like, particle. Michael et al (1995) Gene Therapy 2, 660-668 describes modification of adenovirus to add a cell-selective moiety into a fibre protein. Mutant adenoviruses which replicate selectively in p53-deficient human tumour cells, such as those described in Bischoff et al (1996) Science 274, 373-376 are also useful for delivering the genetic construct of the invention to a cell. Thus, it will be appreciated that a further aspect of the invention provides a virus or virus-like particle comprising a genetic construct of the invention. Other suitable viruses or virus-like particles include HSV, AAV, vaccinia and parvovirus.

A ribozyme capable of cleaving the TAPP polypeptide RNA or DNA may also be useful for reducing expression of the TAPP polypeptide. A gene expressing said ribozyme may be administered in substantially the same and using substantially the same vehicles as for the antisense molecules. Ribozymes which may be encoded in the genomes of the viruses or virus-like particles herein disclosed are described in Cech and Herschlag "Site-specific cleavage of single stranded DNA" US 5,180,818; Altman *et al* "Cleavage of targeted RNA by RNAse P" US 5,168,053, Cantin *et al* "Ribozyme cleavage of HIV-1 RNA" US 5,149,796; Cech *et al* "RNA ribozyme restriction endoribonucleases and methods", US 5,116,742; Been *et al* "RNA ribozyme polymerases, dephosphorylases, restriction endonucleases and methods", US 5,093,246; and Been *et al* "RNA ribozyme polymerases, dephosphorylases, restriction endoribonucleases and methods; cleaves single-stranded RNA at specific site by transesterification", US 4,987,071 .

The genetic constructs of the invention can be prepared using methods well know in the art.

A further aspect of the invention provides a kit of parts useful in carrying out a screening method of the invention comprising TAPP or a polynucleotide encoding TAPP, and PTPL1 or a polynucleotide encoding PTPL1. Preferences for the TAPP and PTPL1 polypeptides are as indicated above in relation to the screening methods of the invention. For example a full length TAPP molecule or fragment thereof comprising the C-terminal PH domain and at least the three, four, five, six, seven, eight, nine or ten most C-terminal amino acids of TAPP (ie residues involved in the interaction with the PTPL1 PDZ domain(s)) may be used. The PTPL1 polypeptide may comprise PDZ domain 1 and/or PDZ domain 5 and may also comprise the phosphatase domain.

A further aspect of the invention provides a method for modulating the cellular activity or location of PTPL1, the method comprising the step of exposing the PTPL1 to a compound that modulates the interaction of PTPL1 with TAPP; or which modulates or mimics the interaction of TAPP with PtdIns(3,4)P₂; or which modulates the cellular location of TAPP, or which modulates the amount or type of TAPP (as discussed above). The method may be performed *in vitro* . It may be performed in cells or in cell free systems, for example using purified components.

A further aspect of the invention provides a method for modulating signalling via Ptdlns(3,4)P₂, the method comprising the step of exposing the PTPL1 to a compound which modulates the interaction between TAPP and PTPL1. The method may be performed *in vitro*. It may be performed in cells or in cell free systems, for example using purified components.

A further aspect of the invention provides the use of a compound that inhibits the interaction of TAPP with PTPL1 in the manufacture of a medicament for treating diabetes (Type II or Type I), inhibition of apoptosis, for example in the treatment of ischaemic disease, wound healing or nerve regeneration.

A further aspect of the invention provides the use of a compound that promotes the interaction of TAPP with PTPL1 in the manufacture of a medicament useful in promoting apoptosis, for example in treating cancer or in the resolution of inflammation. By inflammatory disease is included immune system disorders, for example autoimmune diseases, as will be apparent to those skilled in the art.

The invention is now described in more detail by reference to the following non-limiting Figures and Examples.

### Figure Legends

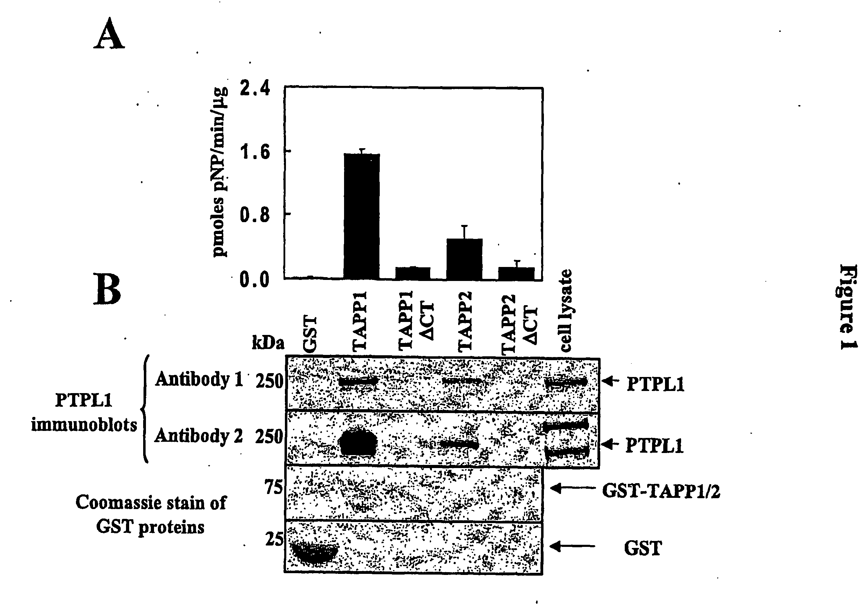

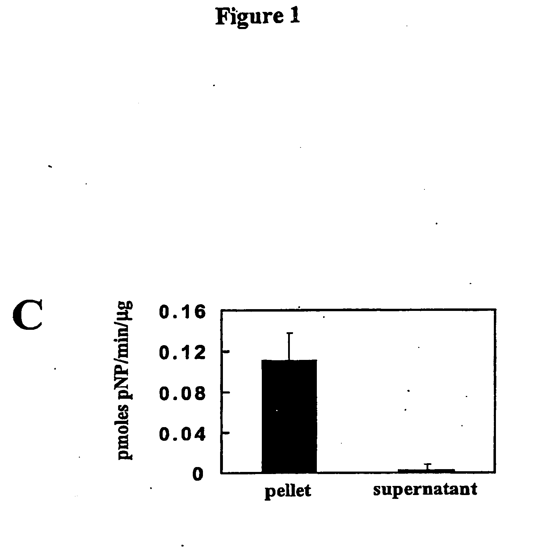

**Figure 1****. Binding of TAPP and TAPP2 to PTPL1.** (A) 293 cells were transiently transfected with DNA constructs encoding for the indicated GST fusion proteins of the wild type and mutant forms of TAPP1 and TAPP2. 36 hours post-transfection cells were lysed and the GST fusion proteins purified by affinity chromatography on glutathione-Sepharose beads. Equal amounts of purified protein were assayed for *p*NPP phosphatase activity. The results are presented as pmoles of pNP produced/min/µg of TAPP protein added to the assay. Results are the average ± SEM of two separate experiments in which each condition was assayed in duplicate. (B) The indicated purified GST fusion proteins were electrophoresed on a 3-8%(w/v) polyacrylamide gel and immunoblotted with two separate anti-PTPL1 antibodies. The GST proteins were also electrophoresed on a 4-12 % (w/v) polyacrylamide gel, which was stained with Coomassie Brilliant Blue to detect expression of GST as well as wild-type and mutant GST-TAPP1 and GST-TAPP2. The position of the molecular-mass markers (Bio-Rad precision markers) is indicated. Similar results were obtained in 3 separate experiments (C) Equal amounts of GST-TAPP1 protein that had been purified from 293 cell lysates as described in (A), were incubated with the "PTPL1 antibody 1" conjugated to Protein G Sepharose and following incubation the *p*NPP phosphatase activity in the supernatant and washed pellet fraction of the immunoprecipitation was measured. The results shown are the average ±SD for a single experiment in which each determination was carried out in triplicate. Similar results were obtained in 3 separate experiments.

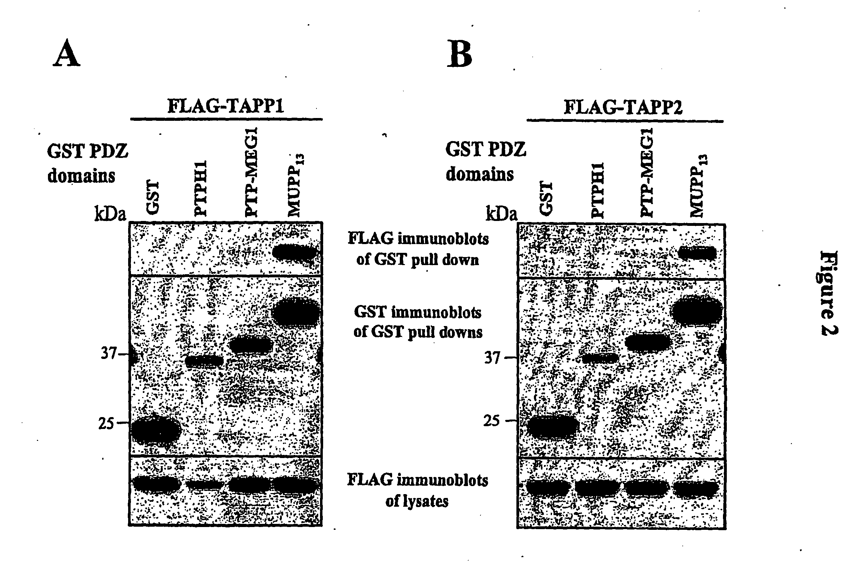

**Figure 2****. TAPP1 does not bind the PDZ domains of PTP-MEG1** or **PTPH1.** 293 cells were co-transfected with plasmids encoding for FLAG-TAPP1 (A) or FLAG-TAPP2 (B) in the presence of constructs encoding for the expression of GST, GST-PTPH1-PDZ domain, GST-PTP-MEG1-PDZ domain or the 13th PDZ domain of MUPP1 (MUPP₁₃). 36 h post transfection, the GST fusion proteins were purified by glutathione-Sepharose and immunoblotted using the anti-FLAG antibody to detect the presence of TAPP1/TAPP2 proteins or with an anti-GST antibody to visualise GST fusion proteins. Similar results were obtained in 2 separate experiments, which were each analysed in duplicate.

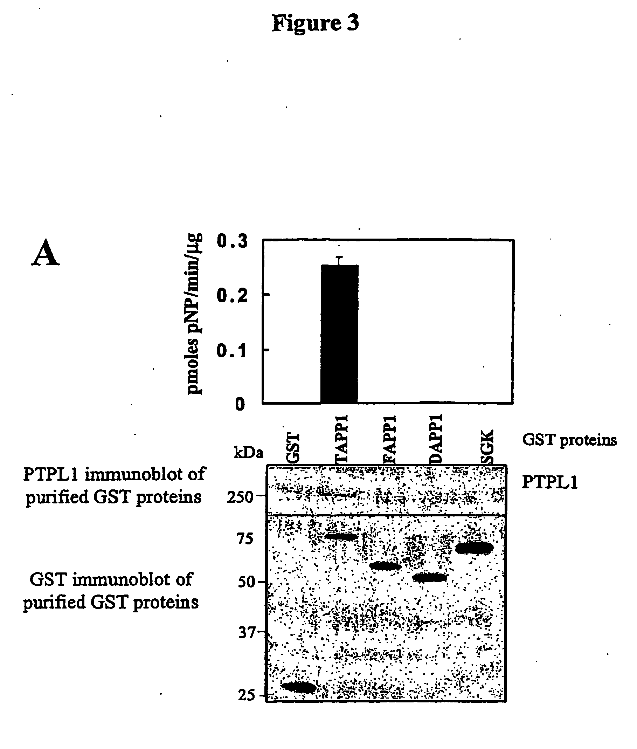

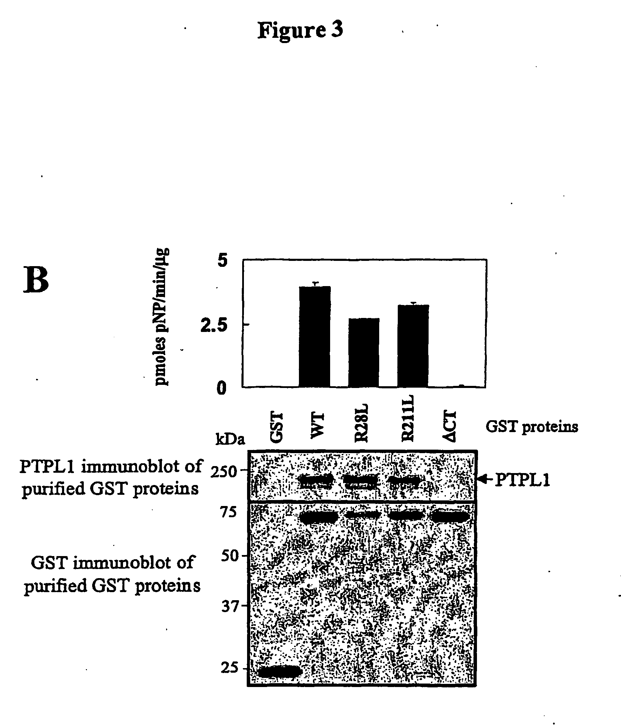

**Figure 3****. PTPL1 binds TAPP1 specifically.** (A) 293 cells were transiently transfected with plasmids encoding DNA for the expression of GST, GST-TAPP1, GST-FAPP1, GST-DAPP1 and GST-SGK. 36 hours post transfection, GST fusion proteins were purified using glutathione-Sepharose and assayed forpNPP phosphatase activity and immunoblotted with an anti-PTPL1 antibody (antibody 1) and an anti-GST antibody. The results are presented as pmoles of pNP produced/min/µg of GST fusion protein added to the assay. pNPP phosphatase activity assays were carried out in triplicate and results are presented as the average ± SD for a single experiment. Similar results were obtained in 2 separate experiments. (B) As above except that 293 cells were transfected with the wild type and indicated mutants of TAPP1.

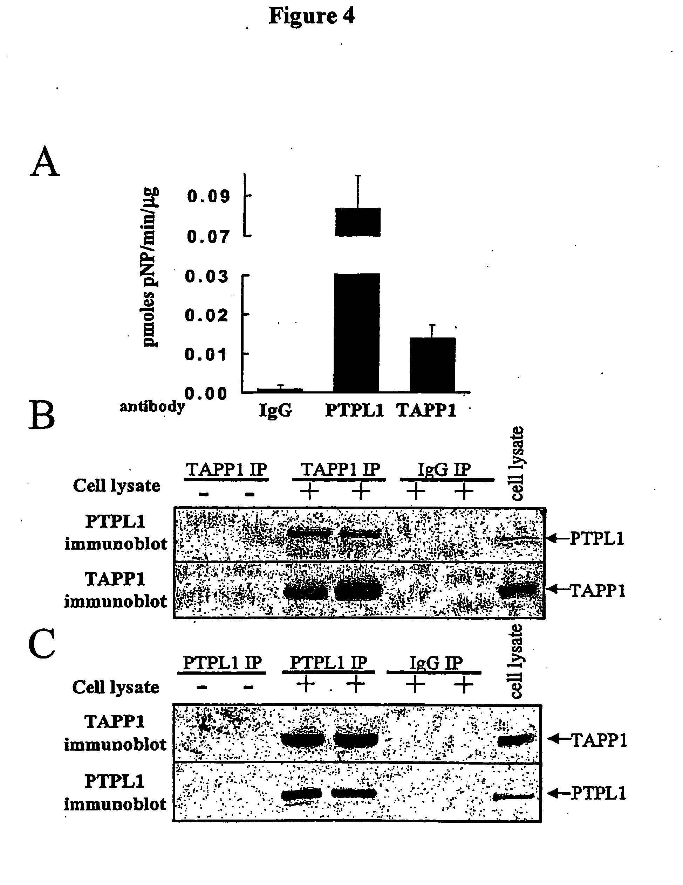

**Figure 4****. Endogenous TAPP1 is associated with PTPL1** (A) TAPP1 or PTPL1 were immunoprecipitated from 2 mg of 293 cell lysate using 10 µg of anti-TAPP1 antibody, 6 µg of anti-PTPL1 antibody (antibody 1), or 10 µg of preimmune IgG, covalently coupled to protein G-Sepharose. The immunoprecipitates were assayed for *p*NPP phosphatase activity (A) or immunoblotted with the indicated antibodies (B and C). The results of the *p*NPP phosphatase activity are presented as pmoles of pNP produced/min/µg of total protein immunoprecipitated from the cell lysate. In each gel, 20 µg of total cell lysate was also immunoblotted in parallel. *p*NPP phosphatase activity assay's were carried out in triplicate and results are presented as the average ±SD for a single experiment. Similar results were obtained in 2 separate experiments.

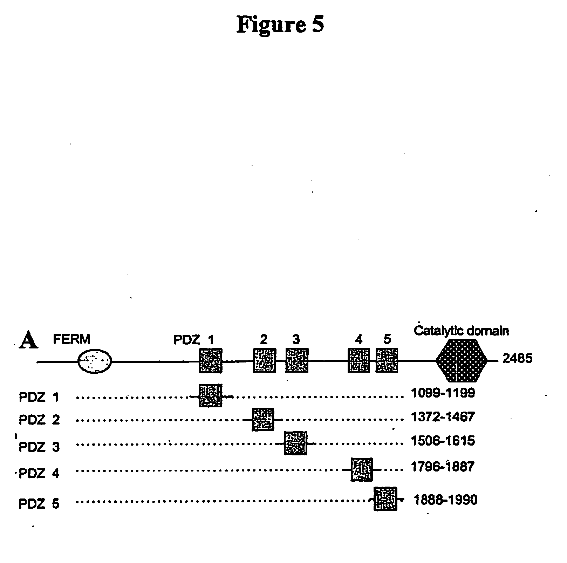

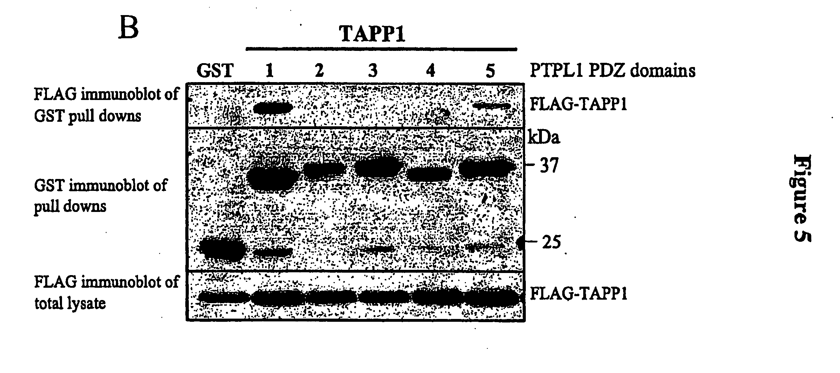

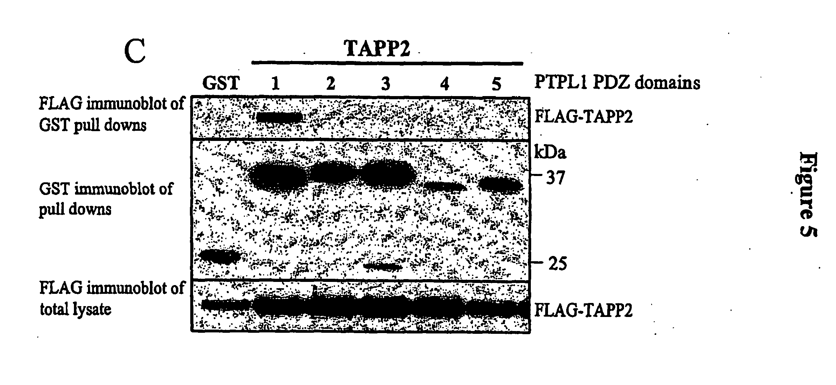

**Figure 5****. TAPP1 and TAPP2 bind to the first PDZ domain of PTPL1**. (A) Schematic diagram of the domains in PTPL1 and those domains expressed in 293 cells. (B) 293 cells were co-transfected with DNA encoding FLAG-TAPP1 (upper panel) or FLAG-TAPP2 (lower panel) together with GST alone or the indicated PTPL1 GST-PDZ domains. 36 hours post transfection, the GST-fusion proteins were affinity purified on glutathione-Sepharose and analysed by immunoblotting with anti-FLAG antibody to detect FLAG-TAPP1 or FLAG-TAPP2 or with an anti-GST antibody to visualise the levels of the GST fusion proteins. Prior to affinity purification cell lysates were also immunoblotted with anti-FLAG antibody to ensure levels of TAPP1 and TAPP2 were similar for each condition. Similar results were obtained in 3 separate experiments.

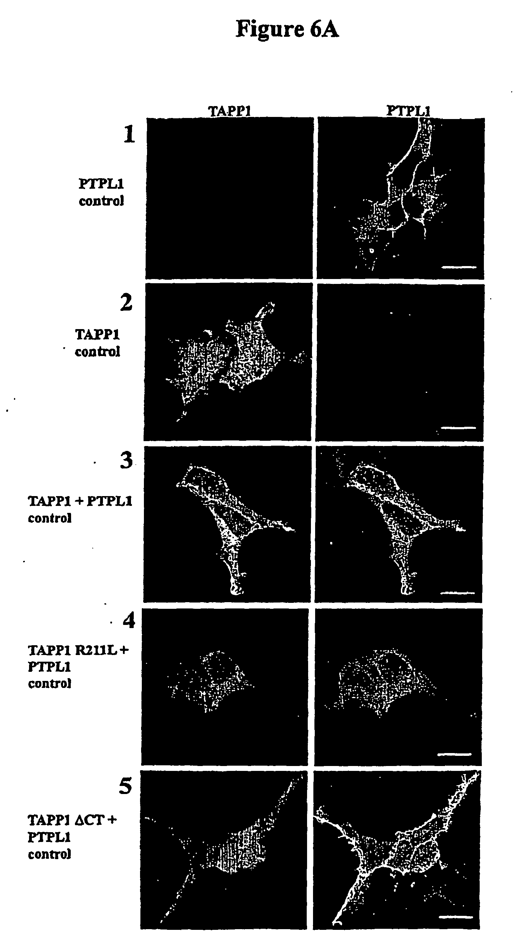

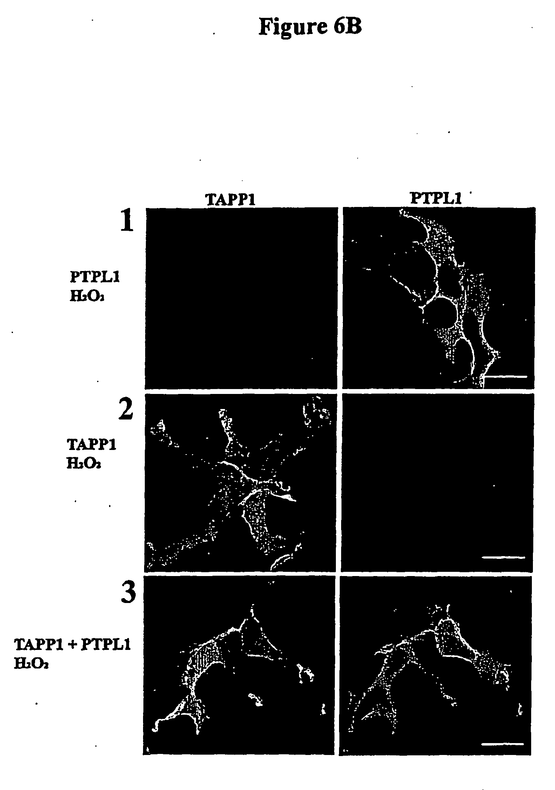

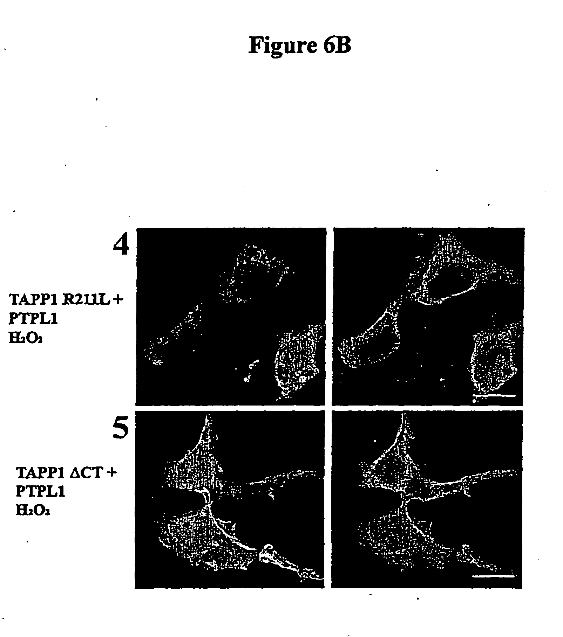

**Figure 6****. TAPP1 anchors PTPL1 in the cytoplasm away from the plasma membrane.** (A + B) 293 cells were transfected with the indicated constructs encoding for the expression of the wild type or indicated mutants of YFP-TAPP1 or HA-PTPL1. 24 h post transfections the cells were deprived of serum for 16 h and either left unstimulated or stimulated with 1 mM H₂O₂ for 10 min. The cells were fixed in 4% (w/v) paraformaldehyde and immunostained with anti-HA antibody to detect PTPL1 (594 Alexa Fluor anti-mouse secondary antibody, red channel) and YFP-TAPP1 localisation was visualised directly through the YFP fluorescence (green channel). Confocal image sections were taken using a Zeiss LSM 510 META confocal microscope. The cells shown are representative images obtained in 3 separate experiments. The scale bars correspond to 10 µm.

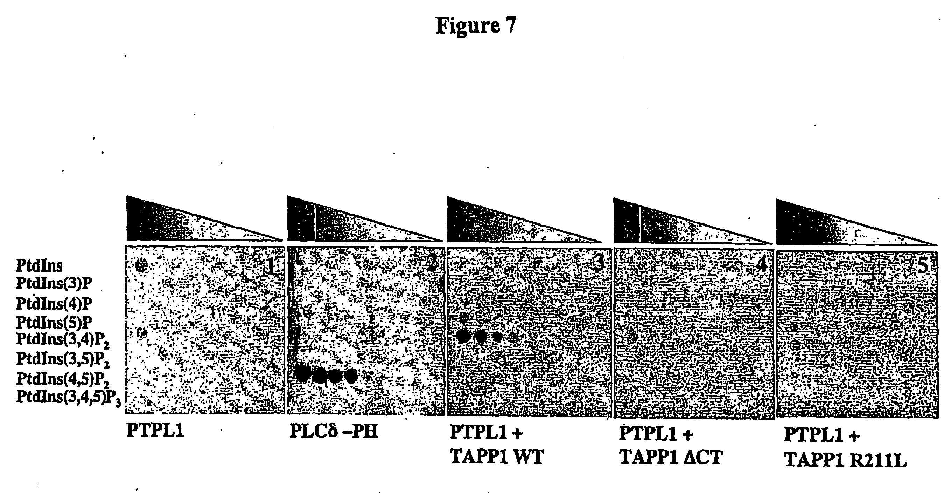

**Figure 7****. TAPP1 localises PTPL1 to PtdIns(3,4)P₂.** The ability of PTPL1 to bind phosphoinositides in the presence or absence of wild type or indicated mutant forms of TAPP1 were analysed using a protein-lipid overlay assay as described in the experimental procedures. Serial dilutions of the indicated phosphoinositides (250 pmol, 100 pmol, 50 pmol, 25 pmol, 12.5 pmol, 6.3 pmol, 3.1 pmol and 1.6 pmol) were spotted onto nitrocellulose membranes, which were then incubated with purified GST-HA-PTPL1 either with or without the indicated forms of TAPP1. The PTPL1 that had bound to the membrane was detected using a HA antibody. As a control the PH domain of GST-PLCδ was used at identical concentrations to PTPL1 (1 nM) and binding was detected using an anti-GST antibody. Results shown are representative of three separate experiments.

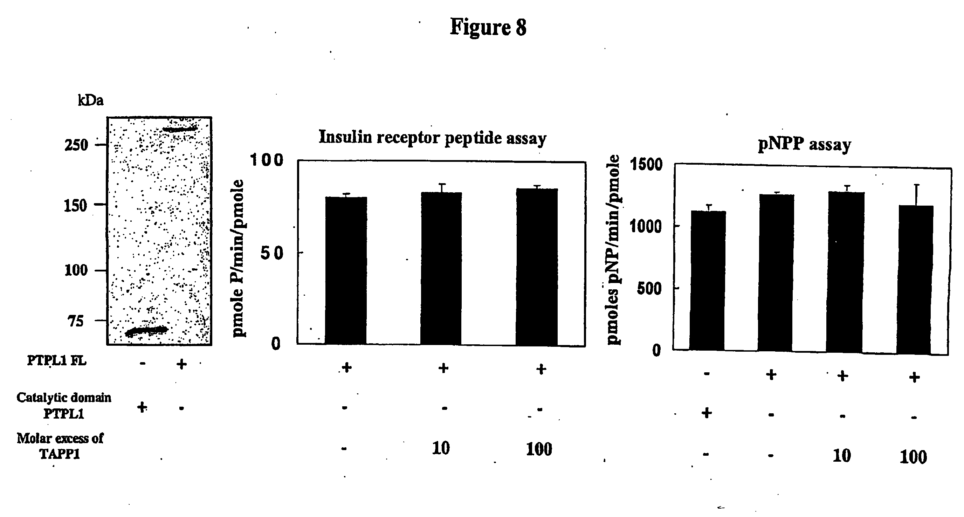

**Figure 8****. Activity of PTPL1 is not influenced by PDZ domains or binding of TAPP1.** 293 cells were transfected with DNA encoding for GST fusion proteins of full length (FL) PTPL1 or a catalytic fragment of PTPL1 that lacks the PDZ domains (residues 2087-2485). 36 hours post transfection, these GST fusion proteins were affinity purified on glutathione-Sepharose. Purified GST-fusion protein was immunoblotted with anti-GST antibody. Prior to assay equal molar amounts of purified phosphatase were incubated in the absence or presence of a 10 fold or 100 fold molar excess of bacterially expressed and purified GST-TAPP 1 for 10 min at room temperature. Assays were initiated by the addition of either *p*NPP or the phosphorylated insulin receptor peptide. The results of the *p*NPP phosphatase activity are presented as pmoles of pNP produced/min/pmole of GST-PTPL1 fusion protein added to the assay and for the insulin receptor peptide assay presented as pmoles of phosphate released/min/pmole of GST-PTPL1 protein added to the assay. Results are presented as the average ± SD of a single experiment with each condition assayed in triplicate. Similar results were obtained in two separate experiments.

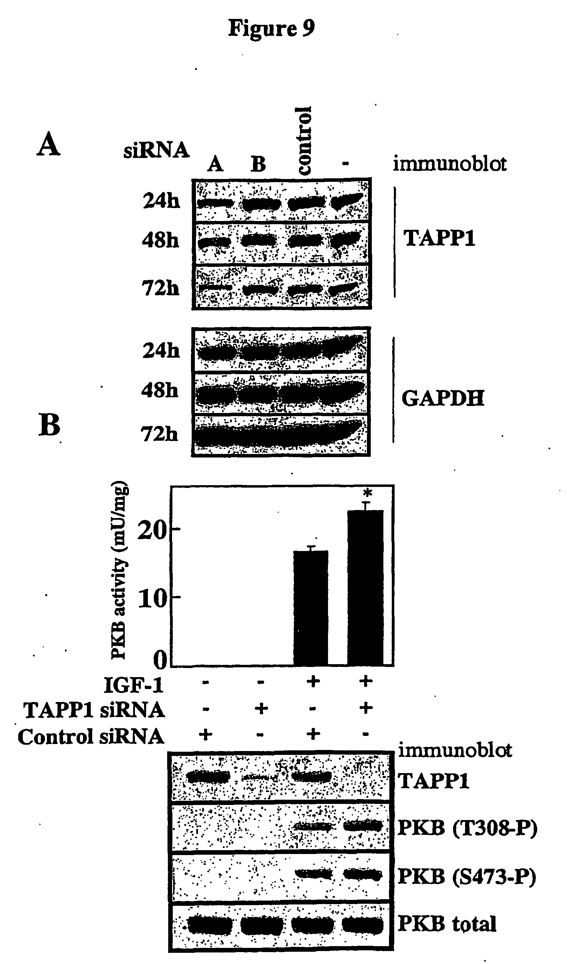

**Figure 9****. Knock-down of TAPP1 moderately enhances PKB activation in response to IGF1.** A) 293 cells were either not transfected or transfected with the indicates TAPP1 RNAi duplexes or a control RNAi duplex. At the indicated times post-transfection, cells were lysed and 20 µg of lysate immunoblotted with the TAPP1 or GAPDH antibodies. (B) as above except that 56 h post-transfection cells were deprived of serum for a further 16 h and left unstimulated or stimulated with 50 ng/ml IGF1 for 5 min. Cells were lysed and PKB immunoprecipitated and assayed using the Crosstide peptide as a substrate. The results are presented as mUnits activity/mg lysate ± SEM where 1 mU represents 1 pmol of substrate phosphorylated in 1 min. Levels of TAPP1, PKB protein and phosphorylation of PKB at T308 and S473 were analysed by immunoblotting with appropriate antibodies. Stimulations were carried out in duplicate with each duplicate sample being assayed in triplicate. Results are representative of two separate experiments. * indicates P<0.005 employing the students t test.

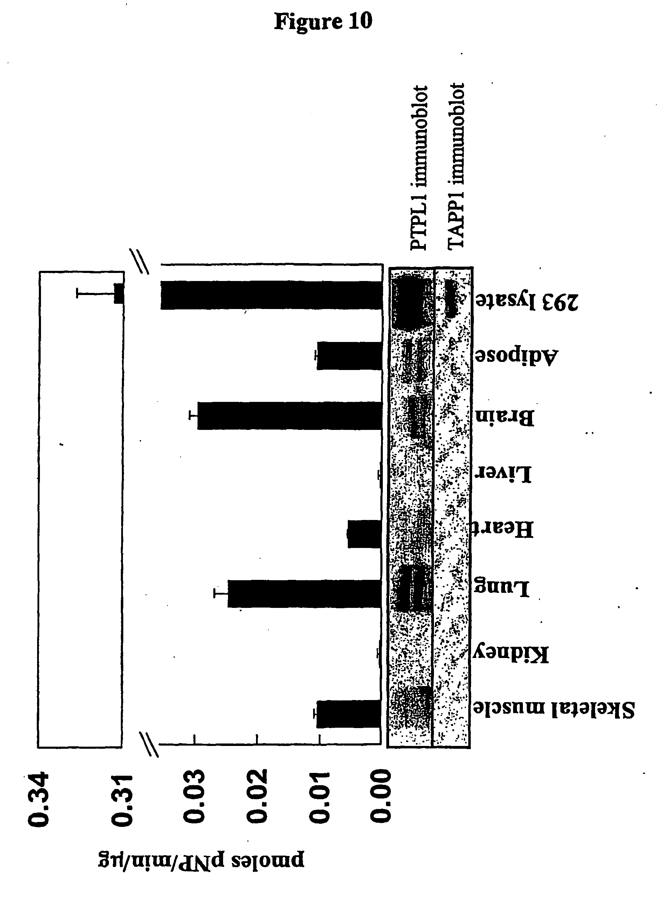

**Figure 10****. Expression of endogenous PTPL1 in mouse tissues.** Mouse tissues and 293 cells were extracted and lysed as described in materials and methods. PTPL1 activity present in each lysate was determined by immunoprecipitation from 1mg of lysate using PTPL1 antibody (antibody 1) and subjecting the washed immunoprecipitates to a pNPP phosphatase assay as described in the materials and methods. Phosphatase activity assays were carried out in triplicate and results are presented as the average ± SEM for a single experiment. Results are presented as pmoles of pNP produced/min/µg of total protein in lysate that was immunoprecipitated and similar results were obtained in two separate experiments. 20µg of each of the indicated lysate was subjected to immunoblot analysis with the anti-PTPL1 or anti-TAPP1 antibody. Similar results were obtained when analysing the levels of PTPL1 and TAPP1 from 2 different mice.

### Example 1: Interaction of the protein tyrosine phosphatase PTPL1 with the PtdIns(3,4)P₂ binding adaptor protein TAPP1

It has been postulated that PtdIns(3,4)P₂, one of the immediate breakdown products of PtdIns(3,4,5)P₃, functions as a signalling molecule in insulin and growth factor stimulated pathways. To date, the TAndem PH domain-containing Protein-1 (TAPP1) and related TAPP2 are still the only known PH domain-containing proteins that interact strongly and specifically with PtdIns(3,4)P₂. In this Example we demonstrate that endogenously expressed TAPP1 is constitutively associated with the Protein Tyrosine Phosphatase Like-Protein-1 (PTPL1 also known as FAP-1). We show that PTPL1 binds to TAPP1 and TAPP2, principally though its first PDZ domain and show that this renders PTPL1 capable of associating with PtdIns(3,4)P₂ *in vitro.* Our data suggest that the binding of TAPP1 to PTPL1 does not influence PTPL1 phosphatase activity, but instead functions to maintain PTPL1 in the cytoplasm. Following stimulation of cells with hydrogen peroxide to induce PtdIns(3,4)P₂ production, PTPL1 complexed to TAPP1, translocates to the plasma membrane. This Example provides the first evidence that TAPP1 and PtdIns(3,4)P₂ could function to regulate the membrane localisation of PTPL1. We conclude that PTPL1 may be recruited to the plasma membrane by increasing levels of PtdIns(3,4)P₂, triggering a negative feedback loop in which PI 3-kinase-dependent or other signalling pathways may be switched off by the phosphatase catalysed dephosphorylation of receptor tyrosine kinases or tyrosine phosphorylated adaptor proteins such as IRS 1 or IRS2. Consistent with this notion, RNAi-mediated knock-down of TAPP1 in 293 cells enhanced activation and phosphorylation of PKB following IGF1 stimulation.

**Abbreviations:** DAPP1, dual adaptor for phosphotyrosine and 3-phosphoinositides; EST, expressed sequence tag; FAP-1, Fas-associated phosphatase-1; FERM Four.1 protein Ezrin Radixin and Moesin; GST, glutathione-S-transferase; IGF, Insulin-like Growth Factor; *p*NPP, para-nitrophenyl phosphate; MUPP1, multi-PDZ-doinain protein-1; PKC, protein kinase C; PDZ, postsynaptic density protein (PSD-95)/Drosophila disc large tumour suppressor (Dlg)/tight junction protein (ZO1); PDK1, 3-phosphoinositide-dependent protein kinase-1; PH, pleckstrin homology; PI 3-kinase phosphoinositide 3-kinase; PKB, protein kinase B; PtdIns, phosphatidylinositol; SHIP, Src homology domain 2-containing inositol 5-phosphatase TAPP, TAndem PH domain containing Protein.

### Experimental

**Materials.** Protease-inhibitor-cocktail tablets were from Roche, foetal bovine serum and other tissue culture reagents were from Bio Whittaker, *para*-nitrophenyl phosphate (pNPP) was from Sigma, glutathione-Sepharose was from Amersham Pharmacia Biotech and BIOMOL Green reagent was from BIOMOL research laboratories. The precast 4-12%(w/v)-polyacrylamide/SDS/Bis-Tris gradient gels, the 3-8%(w/v)-Tris-acetate gels and the 10% Bis-Tris gels were from Invitrogen. GST-TAPP1 was expressed in *E.coli* as described previously [13]. Synthetic RNA duplexes were synthesised by Dharmacon, Lipofectamine 2000 and IGF-1 was from Invitrogen, phosphoinositide derivatives were from Cell Signals, Hybond-C extra was from Amersham Pharmacia. The phosphorylated insulin receptor peptide was synthesized by Graham Bloomberg (Bristol). The GST-PH domain of PLCδ was described previously [13] and was a kind gift of Dr A. Gray.

**Antibodies.** Antibodies raised against the whole TAPP1 protein were described previously [17], antibodies raised against the first 300 amino acids of human PTPL1 (antibody 1) or the 20 C-terminal residues of human PTPL1 (antibody 2) were both purchased from Santa Cruz. Pre-immune IgG antibody was purified from non-immunised sheep serum using a protein G-Sepharose column. Secondary antibodies coupled to horseradish peroxidase used for immunoblotting were from Pierce, monoclonal antibodies recognizing GST, HA and FLAG epitope tags were from Sigma. Phospho-PKB T308 and S473 antibodies were from Cell Signalling, PKB total antibody was raised against whole protein. For immunoprecipitation of PKB for protein kinase assays anti-PKB antibody was used that was raised against the residues 1-149 of human PKB (Upstate). Protein-G coupled to horseradish peroxidase used when immunoblotting of immunoprecipitates was studied was from Bio-Rad. Alexa Fluor conjugated anti-mouse secondary antibody was from Molecular Probes.

**General methods and buffers.** Restriction-enzyme digests, DNA ligations, site-directed mutagenesis and other recombinant DNA procedures were performed using standard protocols. All DNA constructs were verified by DNA sequencing, performed by The Sequencing Service, School of Life Sciences, University of Dundee, Dundee, Scotland, UK, using Big Dye terminator chemistry (Applied Biosystems) on Applied Biosystems automated DNA sequencers. Buffer A contained 50mM Tris/HCl pH7.5, 0.1mM EGTA, 0.27M sucrose and 0.1%(vlv) 2-mercaptoethanol. Buffer B contained 50mM Tris/HCl pH7.5, 0.1mM EGTA and 0.1%(v/v) 2-mercaptoethanol. Buffer C contained 50mM Tris/HCl pH7.5, 1mM EGTA, 1mM EDTA, 1%(w/v) Triton X-100, 1mM Na₃VO₄, 50mM NaF, 5mM sodium pyrophosphate, 0.27M sucrose and 0.1%(v/v) 2-mercaptoethanol and 'complete' proteinase-inhibitor cocktail (one tablet/25ml). Buffer D contained 40mM HEPES pH7.5, 1mM EDTA, 1% (w/v) Triton X-100, 0.27M sucrose, 0.1% (v/v) 2-mercaptoethanol and 'complete' proteinase-inhibitor cocktail (one tablet/25ml). Buffer E contained 3% (w/v) fatty acid-free bovine serum albumin in 50mM Tris/HCl pH7.5, 150mM NaCl and 0.1% (v/v) Tween 20. Sample Buffer contained 50mM Tris/HCl, pH6.8, 2%(w/v) SDS, 10% (v/v) glycerol, 0.005% (w/v) bromophenol blue and 1% (v/v) 2-mercaptoethanol. PtdIns(3,4,5)P₃ phosphatase assays were performed using³³P-labelled PtdIns(3,4,5)P₃ labelled at the 3 position as described previously [20].

**DNA constructs**. Mammalian expression vectors encoding for N-terminal FLAG tagged TAPP1, TAPP1ΔCT (lacks C-terminal 4 amino acids), TAPP1[R28L], TAPP1[R211L], TAPP2 and TAPP2ΔCT (lacks C-terminal 4 amino acids) and that encoding for the 13^{th} PDZ domain of MUPP1 (residues 1885-2042) in the pCMV5 vector or pEBG-2T vector (encodes a . N-terminal glutathione S-transferase GST tag) or pEYFP-C1, (Clonetech), have been described previously [13, 17]. Individual HA tagged PTPL1 PDZ domains were amplified by PCR using a full length human PTPL1 cDNA clone kindly provided by Heldin CH[21]. using the following primers: 1st PDZ domain (residues 1099 to1199) 5'-ggatccgccaccatgtacccatacgatgtgccagattacgccgcaaagtatggcttgggatttcaaatt-3' and 5'-ggatcctcagtagtttttcatctcattggtgagatg-3' 2nd PDZ domain (residues 1372 to 1467) 5'-ggatccgccaccatgtacccatacgatgtgccagattacgccaaaaatgataacagcttggggataagt -3' and 5'-ggatcctcaacactgtggggttaccgggacatgttc-3' 3rd PDZ domain (residues 1506 to 1615) 5'-ggatccgccaccatgtacccatacgatgtgccagattacgccaaaaatagctcaggtctaggattcagt-3' and 5'-ggatcc'tcaactgctgtttggaagtacttgtgctgg-3' 4th PDZ domain (residues 1796 to 1887) 5'-agatctgccaccatgtacccatacgatgtgccagattacgccaaaggaagcctgggttttacagtaacc-3' and 5'-agatcttcacgttagtgttatgtccggtagcaaatg-3' 5th PDZ domain (residues 1888 to 1990) 5'-agatctgccaccatgtacccatacgatgtgccagattacgcctgcaacaaagaggagttgggtttttcc-3 and 5'-agatcttcagtaggaaccattgcctttggttgactt-3'. The PCR products were ligated into pCR2.1 TOPO vector (Invitrogen), sequenced and subcloned into the *Bam*H1 site of pEBG2T [22] and pCMV5 vectors [23] as a *Bam*H1-*Bam*H1 fragment (PDZ 1,2,3) or as a *Bgl*II-*Bgl*II fragment (PDZ 4 and 5). Full length PTPL1 with an N-terminal HA tag was generated by a combination of PCR and subcloning of the PTPL1 cDNA and subcloned into the pCMV5 and pEBG2T vectors. Full length PTPL1 was cloned using the following primers; 5'-caccatcgatgccccatgtacccatacgatgtgccagattacgccatgcacgtgtcactagctgaggccctg g-3'and 5'-ctgggccactagttttgaactggtaagggg-3' to amplify the first 835bp. This PCR product was ligated into pCR2.1 TOPO, sequenced and digested with *Cla*I and *Spe*1. Full length PTPL1 construct mentioned above was digested with *Spe*1 and *Sal*1 and the resulting 7kb fragment was used in a ligation with the first 835bp fragment and ligated into the *Cla*1 *Sal*1 sites of pCMV5 vector [23].. The phosphatase domain of PTPL1 (residues 2087-2485) was amplified using the following primers; 5'-caccggatccgccaccatggactacaaggacgacgatgacaagacattaaagatgaatgggaagttatca gaagaga-3' and 5'- ggatcctcacttcagaagctgaggctgctgtttttgc-3'. The PCR product was ligated into TOPO ENTRD vector (Invitrogen) sequenced and then subcloned into the pEBG2T vector. HA tagged PDZ domain of PTP-MEG1 (NCBI Acc: P29074) covering AA 509-610 was amplified by PCR from an EST (NCBI Acc.N. BI916937, IMAGE clone 5241923) using the following primers: 5'-caccggatccgccaccatgtacccataegatgtgecagattacgccggtggtattccacatgataatettgtc-3' and 5'-ggatcctcatacatcatatacagcattaggtcgaac-3', and subcloned into the pEBG2T vector.. HA tagged PDZ domain of PTPH1 (NCBI Acc: NP_002820) covering residues 519-601 was amplified by PCR from an EST (NCBI Acc.N. BG115423, IMAGE clone 4416683) with the following primers: 5'-agatctgccaccatgtacccatacgatgtgccagattacgccggaaaatttggatttaatcttaaggg-3' and 5'-tcatgagcggacagctctcctcctgat-3', and subcloned into the pEBG2T vector. The PCR products were ligated to pCR 2.1 TOPO vector, sequenced, then subcloned further into pEBG2T mammalian expression vectors as *BamH*1*-BamH*1 or *Bgl*2*-Not*1 fragment.

**Cell culture and cell lysis.** Human embryonic kidney 293 cells were cultured on 10 cm-diameter dishes in Dulbecco's modified Eagle's medium supplemented with 10% (v/v) foetal calf serum. Cells were lysed in ice-cold Buffer C unless otherwise indicated and the lysates centrifuged for 10 min at 16 000g at 4°C. Protein concentrations were determined using the Bradford method and BSA was employed as the standard.

**Transient transfections of 293 cells.** 293 cells cultured on 10 cm diameter dishes were transfected with 5µg of the indicated plasmid, using the modified calcium phosphate method [24]. 36h post-transfection the cells were lysed in 1ml Buffer C unless otherwise indicated and the lysates clarified by centrifugation at 13,000g for 15mins at 4°C. For immunolocalisation studies 293 cells were transfected with FuGene 6 transfection reagent (Roche) following manufacturer's protocol.

**Purification of GST fusion proteins from 293 cell lysates.** For the experiments performed in Figures 1, 3, 7, 8 and 10, the clarified cell lysates derived from twenty 10 cm diameter dishes of 293 cells lysed in Buffer D, were pooled and incubated with 0.25 ml of glutathione-Sepharose for 1h at 4°C. The beads were washed twice in Buffer D followed by two further washes in Buffer A. The GST fusion proteins were eluted by incubating the resin with an equal volume of Buffer A containing 40mM glutathione and the eluted protein was collected after centrifugation through a Spin X (Costar) column. For the experiments performed in Figures 2 and 5 cells were lysed in Buffer C and following affinity purification glutathione-Sepharose beads washed in Buffer C containing 0.5 M NaCl followed by Buffer B.

**Protein Tyrosine Phosphatase assay**. Phosphatase activity was measured by monitoring hydrolysis of para-nitrophenyl phosphate (*p*NPP) using a spectrophotometric assay which detects formation of the reaction product para-nitrophenol by absorbance of 405 nm [25] or monitoring the dephosphorylation of the insulin receptor peptide substrate (TRDIYETDYYRK) phosphorylated at the underlined Tyr residues, equivalent to Tyr 1158, Tyr 1162 and Tyr 1163 on the insulin receptor. For the pNPP assay immunoprecipitates or purified GST fusion proteins (between 5-50 µg) were diluted to 0. 1 ml in 20 mM Tris pH7.4, 150 mM NaCl, 1 mM DTT, 10% (v/v) glycerol, pre-incubated at 30°C with gentle agitation for 5mins before addition of a 0.1 ml solution containing 0.1 M imidazole pH7.5, 40 mM *p*NPP and 20mM DTT. Absorbance was measured at 405nm either continually or at an end point for periods of up to 120 min, using a Versa max absorbance plate reader with Softmax Pro 4.0 software (Molecular Devices). Rates or amounts of para-nitrophenol production were calculated using a standard curve generated from known amounts of para-nitrophenol. For the insulin receptor peptide assay a 50 µl reaction was set up containing 20 mM Tris pH7.4, 150 mM NaCl, 1 mM DTT, 20 µM of the tyrosine-phosphorylated peptide and 100 ng of GST-PTPL1. After 10 min at room temperature, the reactions were terminated by the addition of 0.1 ml of BIOMOL green reagent and after an additional 20 min the amount of phosphate liberated was quantitated by absorbance at 620 nm and measured against inorganic phosphate standards. In all assays we ensured that the rate of para-nitrophenol formation or the dephosphorylation of the insulin receptor peptide, was linear with time.

**Immunoprecipitation of endogenous TAPP1 and PTPL1.** The polyclonal anti-TAPP1 or the pre-immune IgG antibodies were conjugated to Protein G-Sepharose at a ratio of 1 µg of antibody to 1 µl of beads and the commercial PTPL1 antibody 1 at a ratio of 0.5 µg of antibody to 1 µl of beads. These were incubated with 2.5 mg of clarified 293 cell lysate in which the cells had been lysed in Buffer D. After 1h at 4°C with gentle agitation, the immunoprecipitates were washed twice with Buffer D containing 0.5M NaCl, washed twice with Buffer B and either subject to protein tyrosine phosphatase assay and/or to immunoblot analysis. For immunoprecipitation of PTPL1 from mouse tissues, the indicated tissues were extracted from C57BL/6j mice, snap frozen in liquid nitrogen and homogenised to a powder. A 10-fold mass excess of ice-cold Buffer D was added to the powered tissue, that was briefly vortexed and centrifuged at 4°C for 10mins at 13,000g to remove insoluble material. PTPL1 was immunoprecipitated from 1mg of the each of the supernatants as described above. As a control preimmune IgG antibody was also coupled to protein G-Sepharose and also incubated with 1mg of tissue extract. The basal *p*NPP activity associated with the pre-immune immunoprecipitate was subtracted from that observed with the PTPL1 antibody.

**Immunoblotting**. For blots of total cell lysates or tissues 20 µg of protein was used. For the purified GST fusion proteins to detect association of endogenous PTPL1, 10 µg of fusion protein was employed for the PTPL1 blots and 0.1 µg for the GST blots. Samples were subjected to SDS/PAGE and transferred to nitrocellulose. The membranes were blocked in 50mM Tris/HCl (pH7.5, 0.15M NaCl, 0.5% (v/v) Tween (TBS-Tween), 10% (w/v) skimmed milk for 2h, then incubated in the same buffer for 7h at 4°C in the presence of 1 µg/ml of the indicated primary antibody. Detection was performed using the enhanced chemiluminescene (ECL ®; Amersham Pharmacia Biotech) reagent and horseradish peroxidase-conjugated secondary antibodies except for when co-immunoprecipitations were performed where protein G conjugated to horseradish peroxidase was used.

**Immunofluorescence**. 293 cells were cultured on poly-L-lysine coated cover slips to 50% confluence in 6-well plates and transfected with 1µg of a construct encoding wild-type or mutant forms of YFP-TAPP1 in the presence or absence of 1µg of HA-PTPL1 using FuGene 6 transfection reagent (Roche). 24 hours post transfection cells were serum starved for 16 hours. Cells were either left unstimulated or treated with 1mM H₂O₂ for 10mins. Cells were fixed in 4% (w/v) paraformaldehyde in PHEM Buffer (60mM Pipes, 25mM Hepes, 10mM EGTA and 2mM MgSO₄, pH7.0). The cells were washed twice with PBS and permeablised with 0.2% Triton X-100 in PBS for 5mins, washed three times with 0.1% (v/v) PBS-Tween (PBST) before being blocked with 3% fish skin gelatin (FSG) in PBST for 30mins. Cells were then incubated with anti-HA antibody at 2µg/ml in 3% FSG -PBST for one hour, washed three times with PBST and incubated with anti-mouse secondary labelled with Alexa Fluor 594 (Molecular Probes) in 3% FSG/PBST for 30mins. The cells were washed three times with PBST and mounted on slides with Mowiol. The cells were imaged and confocal sections collected, using a Zeiss LSM 510 META confocal microscope. Each channel was scanned independently to avoid cross talk (Multi-Tracking).

**Protein-lipid overlay assay.** In order to assess lipid binding we employed the protein lipid overlay assay that we have described previously [26]. Briefly, 1µl of lipid solution containing 1-250pmol of phospholipids dissolved in a mixture of chloroform/methanol/water (1:2:0.8 by volume) was spotted onto Hybond-C extra membrane and allowed to dry at room temperature for 1hour. The membranes were blocked in Buffer E for 1 hour. 1nM of GST-HA-PTPL1 expressed and purified from 293 cells as described above was preincubated on ice in presence or absence of wild type and mutant forms of 10 nM GST-Flag-TAPP1 also derived from a 293 cell expression described previously [17] in Buffer E. The mixture was then incubated overnight with the Hybond-C membranes at 4°C, with gentle agitation . The membranes were washed 6 times with Buffer E not containing BSA and then incubated with 1 µg/ml anti-HA monoclonal antibody for 2h at room temperature. The membranes were washed again as before, then incubated for 2 hours with 1:5000 dilution of anti-mouse HRP conjugate. Finally the membranes were washed 12 times over 1 hour and the HA-PTPL1 that had bound to the membrane through the interaction of the lipid or through the binding of TAPP1 was detected by enhanced chemiluminescence.

**siRNA studies**. Several RNA duplexes were raised against the human TAPP1 sequence and were selected by the siDESIGN centre programme at http://www.dharmacon.com/. The most effective at reducing endogenous TAPP1 levels possessed the sequence (5'- C CAC CUC AAG AUA GUG CGG-3'). As a control a RNA duplex that does not target any cellular mRNA (5'- G UGC GCA CGU AGA UAG AGU-3') was employed. 293 cells were cultured in 6 well plates to 30% confluence and transfected with 100 pmol of TAPP1 or control RNA duplex per well using Lipofectamine 2000 following the manufacturers protocol. 24h post transfection the cell medium was replaced with fresh DMEM containing 10% fetal calf serum. The cells were left for 32 hours, then deprived of serum for a further 16h. The cells were either left unstimulated or stimulated with 50ng/ml IGF1 for 5 min prior to lysis. PKB activity was determined following its precipitation and assay using the Crosstide peptide as a substrate (GRPRTSSFAEG), as described previously [27].

### Results.

**Evidence that TAPP1 interacts with a protein tyrosine phosphatase.** In order to test the hypothesis that TAPP1 and/or TAPP2 interacted with a PtdIns(3,4,5)P₃ phosphatase and/or a protein tyrosine phosphatase (see introduction), GST-TAPP1 or GST-TAPP2 expressed in 293 cells were affinity purified on glutathione-Sepharose and incubated with either ³³P-PtdIns(3,4,5)P₃ labelled at the 3 position or *para*-nitrophenyl phosphate (*p*NPP) to measure tyrosine phosphatase activity. Although no PtdIns(3,4,5)P₃ phosphatase activity was observed (data not shown), significant *p*NPP phosphatase activity was associated with TAPP1 and to a lesser extent with TAPP2 (Fig 1A).

**Evidence that TAPP1/TAPP2 specifically associate with PTPL1.** We previously found that the four C-terminal amino acids of TAPP1 and TAPP2 comprise a PDZ binding motif, which may enable them to interact with PDZ domain containing protein(s) [17]. We found that TAPP1ΔCT and TAPP2ΔCT mutants, lacking the four C-terminal residues, were associated with far lower levels of pNPP phosphatase compared to the wild type proteins (Fig 1A), indicating that the TAPP-interacting tyrosine phosphatase might contain a PDZ domain. Analysis of protein sequence databases, revealed that only 3 protein tyrosine phosphatases contain PDZ domains, namely PTPL1 [21, 28], PTPH1 [29] and PTP-MEG1 [30]. To test whether PTPL1 was associated with GST-TAPP1 and GST-TAPP2, the GST-TAPP proteins purified from cell extracts on glutathione-Sepharose, were immunoblotted with two commercially available anti-PTPL1 antibodies. We observed that both antibodies recognised a protein of the correct molecular weight for PTPL1 (∼260 kDa), which was associated with GST-TAPP1 and GST-TAPP2, but not the GST-TAPP1ΔCT and GST-TAPP2ΔCT mutants (Fig 1B). As we were unable to obtain antibodies that recognised PTPH1 or PTP-MEG1 in 293 cell lysates, we cloned the single PDZ domain of these tyrosine phosphatases and tested whether they could bind TAPP1 and TAPP2 in a co-transfection binding assay in 293 cells. In Figure 2, we demonstrate that TAPP1 and TAPP2 failed to interact with either the PDZ domain of PTPH1 or PTP-MEG1, whilst in parallel experiments they bound to a PDZ domain of MUPP1, previously shown to bind TAPP 1 and TAPP2 [17].

We next investigated whether the tyrosine phosphatase activity associated with TAPP1 could be immunoprecipitated with an anti-PTPL1 antibody. In Figure 1C, we demonstrate that all detectable tyrosine phosphatase activity associated with GST-TAPP1 was immunoprecipitated with a PTPL1 antibody, suggesting that the bulk of the pNPP tyrosine phosphatase activity associated with TAPP1 can be attributed to PTPL1.

Binding of PTPL1 to TAPP1 and TAPP2 was specific, as no PTPL1 protein or pNPP phosphatase activity was associated with either GST alone or GST fusion proteins of two other PH domains DAPP1 and FAPP1 or the Serum and Glucocorticoid induced protein kinase-1 (SGK1), expressed in 293 cells (Fig 3A). Moreover, consistent with the notion that TAPP1 was interacting with PTPL1 through its C-terminal PDZ domain binding motif, we found that GST-TAPP1 mutants in which either the N-terminal PH domain (TAPP1[R28L]) and C-terminal PH domain (TAPP1[R211L]) were disrupted [13], were still able to interact with endogenously expressed PTPL1, to the same extent as judged by tyrosine phosphatase activity and immunoblot analysis as wild type TAPP1 in 293 cells (Fig 3B).
**Endogenous TAPP1 interacts with endogenous PTPL1.** We next immunoprecipitated endogenous TAPP1 from 293 cells and assessed whether it was associated with *p*NPP phosphatase activity and PTPL1 protein. As a control, immunoprecipitations were performed from the same amount of lysates with preimmune IgG and anti-PTPL1 antibodies. The endogenous TAPP1 immunoprecipitates were associated with significant levels *p*NPP phosphatase, which was absent from the immunoprecipitates obtained using preimmune IgG (Fig 4A). ∼6-fold higher levels of *p*NPP phosphatase activity were immunoprecipitated with the anti-PTPL1 antibody compared with the TAPP1 antibody, suggesting that not all PTPL1 is associated with TAPP1, or that the binding of TAPP1 to PTPL1 suppressed its activity. Immunoblotting studies also confirmed that PTPL1 was efficiently co-immunoprecipitated with the anti-TAPP1 antibody, but not with a preimmune IgG antibody (Fig 4B). Consistent with PTPL1 and TAPP1 being in a complex, endogenous PTPL1 immunoprecipitated from 293 cells, was shown to be associated with TAPP1 (Fig 4C upper panel). In addition, we also stimulated 293 cells with hydrogen peroxide to induce PtdIns(3,4)P₂ production, with only minor PtdIns(3,4,5)P₃ formation [31], but this did not affect the amount of PTPL1 co-purified with TAPP1, indicating that these proteins are constitutively associated (data not shown).
**TAPP1 and TAPP2 interact with the 1^{st} PDZ domain of PTPL1.** PTPL1 is a large protein containing 2485 residues, in which the 5 PDZ domains are located between residues 1102 and 1990, while the phosphatase domain is located at the C-terminus between residues 2087 and 2485 (Fig 5A). As the TAPP1ΔCT and TAPP2ΔCT mutants failed to interact with PTPL1 (Fig 1A and 1B), this indicated that TAPP1 and TAPP2 interacted with one or more of the PDZ domains of PTPL1. Using a co-expression binding assay, we tested the ability of the 5 isolated PDZ domains of PTPL1 to interact with TAPP1 (Fig 5B) and TAPP2 (Fig 5C). We found that the 1^{st} PDZ domain of PTPL1 bound to both TAPP1 and TAPP2, whereas in parallel experiments the 2^{nd}, 3^{rd} and 4^{th} PDZ domains of PTPL1, failed to bind either. We consistently observed that the 5^{th} PDZ domain of PTPL1, interacted weakly with TAPP1, but not TAPP2 (Fig 5B), which might explain why TAPP1 appears to interact more strongly with PTPL1 than TAPP2 (Fig 1A & 1B).
**TAPP1 influences the localisation of PTPL1.** 293 cells were transfected with tagged versions of PTPL1 and/or TAPP1 to enable the localisation of these proteins to be visualised independently in the same cell by confocal fluorescence microscopy. PTPL1 expressed on its own, was located at the plasma membrane and to a lesser extent in the cytoplasm (Fig 6A panel 1), as reported previously [32-34]. The binding of PTPL1 to the plasma membrane has recently been shown to be mediated through a the FERM domain located at the N-terminus of PTPL1 [35]. TAPP1 on its own was localised predominantly in the cytosol of unstimulated cells (Fig 6A panel 2), as reported previously [17, 18]. When PTPL1 and TAPP1 were co-expressed, co-localisation was observed in the cell cytoplasin and significantly lower levels of PTPL1 were found at the plasma membrane compared to PTPL1 expressed alone (Fig 6A Compare panels 1 and 3). Moreover, this effect of TAPP1 on PTPL1 localisation was even more evident when PTPL1 was co-expressed with the non-PtdIns(3,4)P₂ binding TAPP1[R211L] mutant [17] (Fig 6A panel 4). The TAPP1ΔCT mutant, that does not bind PTPL1, failed to induce cytoplasmic localisation of PTPL1 (Fig 6A panel 5). TAPP1 translocates to the plasma membrane in response to stimuli such as H₂O₂ that induce the formation of PtdIns(3,4)P₂ (Fig 6B panel 2), as reported previously [17]. The cellular localisation of PTPL1 expressed alone is not altered by exposure to H₂O₂ (Fig 6B panel 1), while TAPP1 was translocated to the plasma membrane whether or not it was co-expressed with PTPL1 (Fig 6B panels 2 and 3). When PTPL1 was co-expressed with TAPP1 (Fig 6B panel 3), but not mutant TAPP1[R211L] (Fig 6B panel 4), significantly higher levels of PTPL1 were found at the plasma membrane of H₂O₂ stimulated cells compared to unstimulated cells.
**Evidence that TAPP1 enables PTPL1 to associate with PtdIns(3,4)P_{2.}**
As discussed above, the FERM domain of PTPL1 enables it to associate with plasma membranes and it has been suggested that this could be mediated through the binding of the FERM domain to PtdIns(4,5)P₂ [35]. However, the evidence that the FERM domain interacts with PtdIns(4,5)P₂ specifically is weak. Consistent with this employing a protein-lipid overlay assay we were unable to detect any significant interaction of HA epitope tagged purified PTPL1 with any phosphoinositide tested employing a HA antibody to specifically detect PTPL1 (Fig 7, panel 1). As a control we demonstrate that the PH domain of PLCδ in a parallel experiment bound strongly to PtdIns(4,5)P₂ (Fig 7, panel 2). Addition of a 10-fold excess of wild type TAPP1 to PTPL1, resulted in association of PTPL1 with PtdIns(3,4)P₂ (Fig 7, panel 3). TAPP1 in the absence of PTPL1 was not detected bound to PtdIns(3,4)P₂ as it is not HA-tagged and does not cross react with the HA antibody (data not shown). As further controls we demonstrate that a mutant of TAPP1 lacking the C-terminal PDZ-binding motif that can not bind PTPL1 (Fig 1), does not localise PTPL1 to PtdIns(3,4)P₂ (Fig 7. panel 4). Moreover, the non-PtdIns(3,4)P₂-binding TAPP1[R211L] mutant that still interacts with PTPL1 (Fig 3B), also failed to localise PTPL1 to PtdIns(3,4)P₂ (Fig 7, panel 5). These results show that TAPP1 can recruit PTPL1 to PtdIns(3,4)P₂ in vitro.
**TAPP1 does not influence the activity of PTPL1**. We next wished to evaluate whether the PDZ domains of PTPL1 or binding of TAPP1 to PTPL1 influenced the pNPP phosphatase activity or the ability of PTPL1 to dephosphorylate an insulin receptor peptide substrate phosphorylated at residues equivalent to Tyr1158, Tyr1162 and Tyr1163 of the human insulin receptor. In order to do this we expressed in 293 cells either full length GST-PTPL1 or a catalytic fragment lacking all of the PDZ domains (residues 2087-2485). Interestingly, the specific pNPP phosphatase activity of the full length PTPL1 was not significantly different to that of the isolated catalytic domain (Fig 8). Moreover, addition of up to a 100-fold molar excess of TAPP1 to full length PTPL1, did not alter phosphatase activity assessed with either substrate (Fig 8).
**Analysis of PTPL1 expression and activity in different tissues.** The expression of PTPL1 protein and activity in different tissues has not previously been studied. In Figure 10 we demonstrate that significant expression of PTPL1 protein and activity are found in brain, lung, adipose tissue and skeletal muscle with lower levels in heart. We were unable to detect PTPL1 in kidney and liver.
**siRNA depletion of TAPP1 enhances PKB phosphorylation and activation**. To examine the role of TAPP1 in regulating the PI 3-kinase pathway, we employed a specific small interfering RNA (siRNA) approach, by taking advantage of the ability to transfect cells with small RNA duplexes to deplete mRNA and protein levels [36]. We evaluated several siRNA sequences for their ability to reduce TAPP1 protein levels over a 72 h period in 293 cells. One of these sequences (duplex A, Fig 9A), reduced the level of endogenous TAPP1 by 80-90% over a 72 h period, without affecting levels of glyceraldehyde-3-phosphate dehydrogenase (GAPDH). In order to assess how knock-down of TAPP1 might affect a cellular response regulated by PI 3-kinase, we measured activation of PKB employing a quantitative immunoprecipitation kinase assay as well as analysing phosphorylation of its 2 major activating phosphorylation sites (Thr308 and Ser473). Knock-down of TAPP1 did not affect the basal PKB activity or phosphorylation in serum starved cells. However following 5 min of IGF1 stimulation, in comparison to cells transfected with a control RNAi duplex, cells in which TAPP1 expression had been reduced, an increase in phosphorylation of PKBα at Thr308 and Ser473 was observed. This was accompanied by a moderate but nevertheless statistically significant ∼20-30% enhancement of PKB activity.

### Discussion

PTPL1 is a widely expressed [21, 37], large protein tyrosine phosphatase consisting of a FERM domain at the N-terminus, a middle region containing 5 PDZ domains and a tyrosine phosphatase domain at the C-terminus. The central finding of our study is that PTPL1 is associated with TAPP1 and to a lesser extent TAPP2. Our data indicate that the interaction is mediated mainly through the first PDZ domain of PTPL1 binding to the C-terminus of TAPP1/TAPP2. Importantly, we demonstrate that immunoprecipitation of endogenous TAPP1 results in co-immunoprecipitation of PTPL1 and vice versa, suggesting that this interaction is indeed physiological.

Recently, it has been shown that the overexpression of PTPL1 inhibited the activation of PI 3-kinase, in a breast cancer MCF7 cell line, by inducing the dephosphorylation of an insulin receptor substrate protein [38]. In these cells 4-hydroxytamoxifen also induced apoptosis by inhibiting the activation of PI 3-kinase an effect that was blocked by depleting PTPL1 from the cells with an antisense approach [38]. These results provide the first indication that the PI 3-kinase pathway could be a physiological target for the PTPL1 phosphatase. The findings of our study indicate that the binding of PTPL1 to TAPP1/TAPP2 could provide a mechanism by which PI 3-kinase could be down-regulated by PTPL1 and PtdIns(3,4)P₂. We propose that a pool of endogenous PTPL1 is maintained in the cytoplasm of unstimulated cells through its interaction with TAPP1. Following prolonged activation of PI 3-kinase, significant levels of PtdIns(3,4)P₂ accumulate at the membrane leading to the recruitment of the PTPL1-TAPP1 complex to this location. Here PTPL1 could dephosphorylate receptor(s) and/or adaptor protein(s), thereby inducing the inactivation of PI 3-kinase. Thus, PtdIns(3,4)P₂ could trigger the activation of a negative feedback loop to switch off PI 3-kinase. As mentioned in the introduction, some evidence also exists that in vitro TAPP1 may also interact with PtdIns(3,4,5)P₃ albeit more weakly than with PtdIns(3,4)P₂ [12]. Even if TAPP1 bound to PtdIns(3,4,5)P₃ in vivo, this would not compromise its ability to function as a negative inhibitor of the PI 3-kinase pathway once PtdIns(3,4,5)P₃ levels were elevated.

The finding that siRNA-mediated reduction in the expression of TAPP1 in 293 cells moderately enhanced IGF1 mediated phosphorylation and activation of PKB supports the role that TAPP1 exerts a negative influence on the PI 3-kinase pathway (Fig 9). PKB activation and phosphorylation is only moderately enhanced in TAPP1-knockdown cells following IGF1 stimulation. It is possible that the remaining 10-20% levels of TAPP1 expression and/or expression of TAPP2 could be sufficient to maintain suppression of the PI 3-kinase pathway in cells.

Recent work has shown that the FERM domain of PTPL1 plays a crucial role in localising full length PTPL1 to the plasma membrane which was suggested to be mediated through PtdIns(4,5)P₂ binding [35]. However, employing the same protein lipid overlay assay that was used previously to assess binding of PTPL1 to phosphoinositides [35], we were unable to demonstrate a significant interaction of full length wild type PTPL1 with PtdIns(4,5)P₂ (Fig 7, panel 1). Significantly we find that the interaction of PTPL1 with TAPP1 inhibited the constitutive membrane localisation of PTPL1, indicating that TAPP1 may sequester PTPL1 in the cytoplasm. This would keep PTPL1 away from the plasma membrane, which might otherwise dephosphorylate receptor and adaptor proteins to prevent activation of one or more components of signal transduction pathways. The mechanism by which TAPP1 sequesters PTPL1 in the cytosol requires further investigation. Following H₂O₂ treatment of cells the levels of both PTPL1 and TAPP1 are recruited to the plasma membrane where PtdIns(3,4)P₂ would be located (Fig 6B). Consistent with this, we demonstrate that in vitro, PTPL1 complexed to TAPP1 can localise with PtdIns(3,4)P₂ spotted on to a membrane surface (Fig 7).

There is significant evidence that the PTP1B tyrosine phosphatase participates in the dephosphorylation of insulin receptor and therefore negatively regulates the insulin signalling pathway (reviewed in [39]). This is also confirmed by the finding that mice lacking PTP1B are significantly sensitised to insulin [40]. These discoveries have stimulated significant interest in developing inhibitors of PTP1B for the treatment of type 2 diabetes [41]. As in tissues of mice lacking PTP1B, the insulin signalling pathway is still switched off following removal of insulin, other tyrosine phosphatases are also likely to be involved in dephosphorylating insulin receptor and IRS proteins [39]. The identity of these enzymes is unknown, but our results indicate that PTPL1 could play a role in negatively regulating the insulin-signalling pathway. If this can be substantiated by further research, it would indicate that inhibitors of PTPL1 or drugs that prevent PTPL1 from binding to TAPP1, could function to potentiate insulin-signalling pathways and could thus have therapeutic potential for the treatment of diabetes.

To our knowledge, the present results are the first to show that full length PTPL1 possesses similar *p*NPP phosphatase activity to the isolated catalytic domain and we also demonstrate that the binding of TAPP1 to PTPL1 does not influence catalytic activity (Fig 8). This conflicts with the report of Nakai and colleagues [42], who claimed that the isolated catalytic domain of PTPL1 was 100-fold more active than the full length protein. However, these authors did not show control experiments to establish that similar levels of full length PTPL1 and catalytic domain were present in their assays. Moreover, in these studies, the full length PTPL1 was expressed in 293 cells, whereas the catalytic domain was expressed in *E.coli* [42]. Although our results indicate that the PDZ domains of PTPL1 do not influence the catalytic activity of PTPL1 directly, it is possible that they could play a key role in regulating the localisation of PTPL1 and/or control its interaction with cellular substrates. PTPL1 has been reported to interact with numerous proteins through its PDZ domains including the Fas receptor [28, 43], neurotrophin receptor [34], a bromodomain containing protein BP75 [37], the adenomatous polyposis coli-protein [44], the transcription inhibitory protein IκBα [42, 45], PKC Related Kinase-2 [46] and the GTPase-activating protein PARG1 [47]. With the exception of Fas, these interactions have not been confirmed by co-immunoprecipitation of endogenous PTPL1 and the functional relevance of these interactions has not been defined. Our data indicates that TAPP1 is not phosphorylated on tyrosine residues in cells, as it is not recognised by anti-phosphotyrosine antibodies when expressed in unstimulated, IGF1 or pervanadate treated cells (WAK unpublished data). Our data suggest that TAPP1 is more likely to function as a regulator of the cellular localisation of PTPL1, allowing PTPL1 to translocate to the plasma membrane when the levels of PtdIns(3,4)P₂ are elevated.

### References

1 Vanhaesebroeck et al (2001) Synthesis and Function of 3-Phosphorylated Inositol Lipids. Annu Rev Biochem. 70, 535-602.
2 Lemmon, M. A. and Ferguson, K. M. (2000) Signal-dependent membrane targeting by pleckstrin homology (PH) domains. Biochem J. 350, 1-18
3 Leslie et al (2001) Phosphoinositide-regulated kinases and phosphoinositde phosphatases. Chem Reviews. 101, 2365-80
4 Stephens, L et al (2002) Roles of PI3Ks in leukocyte chemotaxis and phagocytosis. Curr Opin Cell Biol. 14, 203-13.
5 Gray, A et al (2003) Nonradioactive methods for the assay of phosphoinositide 3-kinases and phosphoinositide phosphatases and selective detection of signaling lipids in cell and tissue extracts. Anal Biochem. 313, 234-45
6 Banfic, H et al (1998) A novel integrin-activated pathway forms PKB/Akt-stimulatory phosphatidylinositol 3,4-bisphosphate via phosphatidylinositol 3- phosphate in platelets. J Biol Chem. 273, 13-6
7 James, S. R et al (1996) Specific binding of the Akt-1 protein kinase to phosphatidylinositol 3,4,5-trisphosphate without subsequent activation. Biochem J. 315, 709-13
8 Currie, R. A et al (1999) Role of phosphatidylinositol 3,4,5-trisphosphate in regulating the activity and localization of 3-phosphoinositide-dependent protein kinase-1. Biochem J. 337, 575-83
9 Stephens, L et al (1998) Protein kinase B kinases that mediate phosphatidylinositol 3,4,5- trisphosphate-dependent activation of protein kinase B. Science. 279, 710-4
10 Dowler, S et al (1999) DAPP1: a dual adaptor for phosphotyrosine and 3-phosphoinositides. Biochem J. 342, 7-12
11 Thomas, C. C et al (2002) High-resolution structure of the pleckstrin homology domain of protein kinase b/akt bound to phosphatidylinositol (3,4,5)-trisphosphate. Curr Biol. 12, 1256-62
12 Ferguson, K. M et al (2000) Structural basis for discrimination of 3-phosphoinositides by pleckstrin homology domains. Mol Cell. 6, 373-84.
13 Dowler, S et al (2000) Identification of pleckstrin-homology-domain-containing proteins with novel phosphoinositide-binding specificities. Biochem J. 351, 19-31
14 Kanai, F et al (2001) The PX domains of p47phox and p40phox bind to lipid products of PI(3)K. Nat Cell Biol. 3, 675-8.
15 Karathanassis, D et al (2002) Binding of the PX domain of p47(phox) to phosphatidylinositol 3,4-bisphosphate and phosphatidic acid is masked by an intramolecular interaction. Embo J. 21, 5057-68.
16 Thomas, C. C et al (2001) Crystal structure of the phosphatidylinositol 3,4-bisphosphate-binding pleckstrin homology (PH) domain of tandem PH-domain-containing protein 1 (TAPP1): molecular basis of lipid specificity. Biochem J. 358, 287-94.
17 Kimber; W. A et al (2002) Evidence that the tandem-pleckstrin-homology-domain-containing protein TAPP1 interacts with Ptd(3,4)P2 and the multi-PDZ-domain-containing protein MUPP1 in vivo. Biochem J. 361, 525-36.
18 Marshall, A. J. et al (2002) TAPP1 and TAPP2 are targets of phosphatidylinositol 3-kinase signaling in B cells: sustained plasma membrane recruitment triggered by the B-cell antigen receptor. Mol Cell Biol. 22, 5479-91
19 Freeburn, R. W et al (2002) Evidence that SHIP-1 contributes to phosphatidylinositol 3,4,5-trisphosphate metabolism in T lymphocytes and can regulate novel phosphoinositide 3-kinase effectors. J Immunol. 169, 5441-50
20 Walker, S. M et al (2001) TPIP: a novel phosphoinositide 3-phosphatase. Biochem J. 360, 277-83
21 Saras, J et al (1994) Cloning and characterization of PTPL1, a protein tyrosine phosphatase with similarities to cytoskeletal-associated proteins. J Biol Chem. 269, 24082-9.
22 Sanchez, I et al (1994) Role of SAPK/ERK kinase-1 in the stress-activated pathway regulating transcription factor c-Jun. Nature. 372, 794-8
23 Anderson, S et al (1989) Cloning, structure, and expression of the mitochondrial cytochrome-P- 450 sterol 26-hydroxylase, a bile-acid D biosynthetic enzyme. J. Biol. Chem. 264, 8222-8229
24 Alessi, D. R et al (1996) Molecular basis for the substrate specificity of protein kinase B; comparison with MAPKAP kinase-1 and p70 S6 kinase. FEBS Lett. 399, 333-8
25 Dickinson, R. J et al (2002) Characterization of a murine gene encoding a developmentally regulated cytoplasmic dual-specificity mitogen-activated protein kinase phosphatase. Biochem J. 364, 145-55
26 Dowler, S et al (2002) Protein lipid overlay assay. Sci STKE. 2002, PL6
27 Collins, B. J et al (2003) In vivo role of the PIF-binding docking site of PDK1 defined by knock-in mutation. EMBO J. 22, 4202-11.
28 Sato, T et al (1995) FAP-1: a protein tyrosine phosphatase that associates with Fas. Science. 268, 411-5.
29 Yang, Q. and Tonks, N. K. (1991) Isolation of a cDNA clone encoding a human protein-tyrosine phosphatase with homology to the cytoskeletal-associated proteins band 4.1, ezrin, and talin. Proc Natl Acad Sci U S A. 88, 5949-53
30 Gu, M. X et al (1991) Identification, cloning, and expression of a cytosolic megakaryocyte protein-tyrosine-phosphatase with sequence homology to cytoskeletal protein 4.1. Proc Natl Acad Sci U S A. 88, 5867-71
31 Van der Kaay, J et al (1999) Distinct phosphatidylinositol 3-kinase lipid products accumulate upon oxidative and osmotic stress and lead to different cellular responses. J Biol Chem. 274, 35963-8
32 Cuppen, E et al (1998) PDZ motifs in PTP-BL and RIL bind to internal protein segments in the LIM domain protein RIL. Mol Biol Cell. 9, 671-83
33 Cuppen, E et al (1999) A FERM domain governs apical confinement of PTP-BL in epithelial cells. J Cell Sci. 112 ( Pt 19), 3299-308
34 Irie, S et al (1999) Functional interaction of Fas-associated phosphatase-1 (FAP-1) with p75(NTR) and their effect on NF-kappaB activation. FEBS Lett. 460, 191-8
35 Bompard, G et al (2003) Membrane targeting of protein tyrosine phosphatase PTPL1 through its FERM domain via binding to phosphatidylinositol 4,5-biphosphate. J Cell Sci. 116, 2519-30
36 Elbashir, S. M et al (2001) Duplexes of 21-nucleotide RNAs mediate RNA interference in cultured mammalian cells. Nature. 411, 494-8
37 Cuppen, E et al (1999) Identification and molecular characterization of BP75, a novel bromodomain-containing protein. FEBS Lett. 459, 291-8
38 Bompard, G et al (2002) Protein-tyrosine phosphatase PTPL1/FAP-1 triggers apoptosis in human breast cancer cells. J Biol Chem. 277, 47861-9
39 Cheng, A et al (2002) Coordinated action of protein tyrosine phosphatases in insulin signal transduction. Eur J Biochem. 269, 1050-9.
40 Elchebly, M et al (1999) Increased insulin sensitivity and obesity resistance in mice lacking the protein tyrosine phosphatase-1B gene. Science. 283, 1544-8.
41 Zhang, Z. Y. and Lee, S. Y. (2003) PTP1B inhibitors as potential therapeutics in the treatment of type 2 diabetes and obesity. Expert Opin Investig Drugs. 12, 223-33
42 Nakai, Y et al (2000) Identification of IkappaBalpha as a substrate of Fas-associated phosphatase-1. Eur J Biochem. 267, 7170-5
43 Ivanov, V. N et al (2003) FAP-1 association with Fas (Apo-1) inhibits Fas expression on the cell surface. Mol Cell Biol. 23, 3623-35
44 Erdmann, K. S et al (2000) The Adenomatous Polyposis Coli-protein (APC) interacts with the protein tyrosine phosphatase PTP-BL via an alternatively spliced PDZ domain. Oncogene. 19, 3 894-901
45 Maekawa, K et al (1999) Association of protein-tyrosine phosphatase PTP-BAS with the transcription-factor-inhibitory protein IkappaBalpha through interaction between the PDZ1 domain and ankyrin repeats. Biochem J. 337 ( Pt 2), 179-84
46 Gross, C et al (2001) The protein kinase C-related kinase PRK2 interacts with the protein tyrosine phosphatase PTP-BL via a novel PDZ domain binding motif. FEBS Lett. 496, 101-4
47 Saras, J et al (1997) A novel GTPase-activating protein for Rho interacts with a PDZ domain of the protein-tyrosine phosphatase PTPL1. J Biol Chem. 272, 24333-8

## Claims

1. A method for identifying a compound for modulating the cellular activity or location of PTPL1, comprising the step of identifying a compound that modulates the interaction of PTPL1 with TAPP; or which modulates or mimics the interaction of TAPP with PtdIns(3,4)P₂; or which modulates the cellular location of TAPP.

2. The method of claim 1 wherein the method comprises the step of using molecular modelling means to select or design a compound that is predicted to interact with the phosphoinositide binding domain of TAPP, wherein a three-dimensional structure of at least a part of the phosphoinoisitide binding domain of the TAPP is compared with a three-dimensional structure of a compound, and a compound that is predicted to interact with the said phosphoinositide binding domain is selected.

3. The method of claim 2 wherein the three-dimensional structure of at least a part of the phosphoinositide binding domain of the TAPP is a three-dimensional structure of at least a part of the phosphoinositide binding site of the TAPP and a compound that is predicted to interact with the said hosphoinositide binding site is selected.

4. The method of claim 1 wherein the method comprises the steps of exposing a TAPP or PTPL1 polypeptide to a test compound; and determining whether the test compound modulates the interaction of PTPL1 with TAPP; or modulates or mimics the interaction of TAPP with PtdIns(3,4)P₂; or modulates the cellular location of TAPP.

5. The method of claim 1 or 4 wherein the TAPP comprises the C-terminal PH domain of human TAPP1 or TAPP2 and the three or four most C-terminal residues of full length human TAPP1 or TAPP2; and wherein the PTPL1 comprises the PDZ1 and/or PDZ5 domain of human PTPL1 and optionally also the phosphatase domain of human PTPL1.

6. The method of any one of claims 1, 4 or 5 comprising the step of using a fluorescence-based assay system.

7. A method for selecting a compound for modulating signalling via PtdIns(3,4)P₂, the method comprising the step of identifying a compound which modulates the interaction between TAPP and PTPL1.

8. The method of claim 7 wherein the method comprises the steps of exposing a TAPP or PTPL1 polypeptide to a test compound; and determining whether the test compound modulates the interaction between TAPP and PTPL1.

9. A kit of parts useful in carrying out a screening method of the invention comprising TAPP or a polynucleotide encoding TAPP, and PTPL1 or a polynucleotide encoding PTPL1.

10. A method for modulating the cellular activity or location of PTPL1, the method comprising the step of exposing the PTPL1 to a compound that modulates the interaction of PTPL1 with TAPP; or that modulates or mimics the interaction of TAPP with PtdIns(3,4)P₂; or that modulates the cellular location of TAPP wherein the method is performed *in vitro*. and wherein the compound is a compound that inhibits the interaction of TAPP with PTPLI wherein the compound is an SiRNA, RNAi, antisense molecule or ribozyme directed against a polynucleotide encoding TAPP; or wherein the compound is a compound that promotes the interaction of TAPP with PTPLI wherein the compound is a molecule encoding full length native TAPP.

11. A method for modulating signalling via PtdIns(3,4)P₂, the method comprising the step of exposing the PTPL1 to a compound which modulates the interaction between TAPP and PTPL1, wherein the method is performed *in vitro*. and wherein the compound is a compound that inhibits the interaction of TAPP with PTPLI wherein the compound is an SiRNA, RNAi, antisense molecule or ribozyme directed against a polynucleotide encoding TAPP; or wherein the compound is a compound that promotes the interaction of TAPP with PTPLI wherein the compound is a molecule encoding full length native TAPP.

12. The use of a compound that inhibits the interaction of TAPP with . PTPL1 in the manufacture of a medicament for treating diabetes, inhibition of apoptosis, treatment of ischaemic disease, wound healing or nerve regeneration wherein the compound is an siRNA, RNAi, antisense molecule or ribozyme directed against a polynucleotide encoding TAPP.

13. The use of a compound that promotes the interaction of TAPP with PTPL1 in the manufacture of a medicament useful in promoting apoptosis, for example in treating cancer or in the resolution of inflammation wherein the compound is a molecule encoding full length native TAPP.

## Patentansprüche

1. Verfahren zum identifizieren einer Verbindung für das Modulieren der Zellaktivität oder Zelllokalisierung von PTPL1, mit den Schritten des Identifizierens einer Verbindung, die die Wechselwirkung von PTPL1 mit TAPP moduliert oder die die Wechselwirkung von TAPP mit PtdIns(3,4)P₂ moduliert oder imitiert.

2. Verfahren nach Anspruch 1, wobei das Verfahren den Schritt des Verwendens von Molekülmodelliermitten aufweist, um eine Verbindung auszuwählen oder zu konstruieren, von der vorhergesagt wird, dass sie mit der Phosphoinositid-bindenden Domäne von TAPP wechselwirkt, wobei eine dreidimensionale Struktur von mindestens einem Teil der Phosphoinositid-bindenden Domäne des TAPP mit einer dreidimensionalen Struktur einer Verbindung verglichen wird und eine Verbindung, von der vorhergesagt wird, dass sie mit der Phosphoinositid-bindenden Domäne wechselwirkt, ausgewählt wird.

3. Verfahren nach Anspruch 2, wobei die dreidimensionale Struktur mindestens eines Teils der Phosphoinositid-bindenden Domäne des TAPP eine dreidimensionale Struktur mindestens eines Teils des Phosphoinositidbindungsplatzes von TAPP ist und eine Verbindung ausgewählt wird, von der vorhergesagt wird, dass sie mit dem Phosphoinositidbindungsplatz wechselwirkt.

4. Verfahren nach Anspruch 1, wobei das Verfahren die Schritte des Exponierens eines TAPP- oder PTPL1-Polypeptits gegenüber einer Testverbindung und das Bestimmen aufweist, ob die Testverbindung die Wechselwirkung von PTPL1 mit TAPP moduliert oder die Wechselwirkung von TAPP mit PtdIns(3,4)P₂ moduliert oder imitiert oder die Zelllokalisierung von TAPP moduliert.

5. Verfahren nach Anspruch 1 oder 4, wobei das TAPP die C-terminale pH-Domäne von humanem TAPP1 oder TAPP2 und die drei oder vier am weitesten C-terminal liegenden Reste von humanem TAPP1 oder TAPP2 voller Länge aufweist und wobei die PTPL1 die PDZ1- und/oder PDZ5-Domäne von humaner PTPL1 und optional auch die Phosphatasedomäne von humaner PTPL1 aufweist.

6. Verfahren nach einem der Ansprüche 1, 4 oder 5, das den Schritt des Verwendens eines fluoreszenzbasierten Testsystems aufweist.

7. Verfahren zum Auswählen einer Verbindung zum Modulieren der Signalgebung über PtdIns(3,4)P₂, wobei das Verfahren den Schritt des Identifizierens einer Verbindung aufweist, die die Wechselwirkung zwischen TAPP und PTPL1 moduliert.

8. Verfahren nach Anspruch 7, wobei das Verfahren die Schritte des Exponierens eines TAPP- oder PTPL1-Polypeptids gegenüber einer Testverbindung und das Bestimmen aufweist, ob die Testverbindung die Wechselwirkung zwischen TAPP und PTPL1 moduliert.

9. Teilesatz verwendbar beim Durchführen eines erfindungsgemäßen Screening-Verfahrens mit TAPP oder einem TAPP-kodierenden Polynukleotid und PTPL1 oder einem PTPL1-kodierenden Polynukleotid.

10. Verfahren zum Modulieren der Zellaktivität oder Lokalisierung von PTPL1, wobei das Verfahren den Schritt des Exponierens der PTPL1 gegenüber einer Verbindung aufweist, die die Wechselwirkung von PTPL1 mit TAPP moduliert oder die die Wechselwirkung von TAPP mit PtdIns(3,4)P₂ moduliert oder imitiert oder die die Zelllokalisierung von TAPP moduliert, wobei das Verfahren in vitro durchgeführt wird und wobei die Verbindung eine Verbindung ist, die die Wechselwirkung von TAPP mit PTPL1 hemmt, wobei die Verbindung eine siRNA, eine RNAi, ein Antisense-Molekül oder ein Ribozym ist, die/das gegen ein TAPP kodierendes Polynukleotid gerichtet ist, oder wobei die Verbindung eine Verbindung ist, die die Wechselwirkung von TAPP mit PTPL1 fördert, wobei die Verbindung ein Molekül ist, das natives TAPP voller Länge kodiert.

11. Verfahren zum Modulieren der Signalgebung über PtdIns(3,4)P₂, wobei das Verfahren den Schritt des Exponierens der PTPL1 gegenüber einer Verbindung aufweist, die die Wechselwirkung zwischen TAPP und PTPL1 moduliert, wobei das Verfahren in vitro durchgeführt wird und wobei die Verbindung eine Verbindung ist, die die Wechselwirkung von TAPP mit PTPL1 hemmt, wobei die Verbindung eine siRNA, eine RNAi, ein Antisense-Molekül oder ein Ribozym ist, die/das gegen ein TAPP kodierendes Polynukleotid gerichtet ist, oder wobei die Verbindung eine Verbindung ist, die die Wechselwirkung von TAPP mit PTPL1 fördert, wobei die Verbindung ein Molekül ist, das natives TAPP voller Länge kodiert.

12. Verwendung einer Verbindung, die die Wechselwirkung von TAPP mit PTPL1 hemmt, bei der Herstellung eines Medikaments zum Behandeln von Diabetes, der Hemmung von Apoptose, der Behandlung von ischaemischer Erkrankung, für Wundheilung oder Nervenregeneration, wobei die Verbindung eine siRNA, eine RNAi, ein Antisens-Molekül oder ein Ribozym ist, die/das gegen ein TAPP kodierendes Polynukleotid gerichtet ist.

13. Verwendung einer Verbindung, die die Wechselwirkung von TAPP mit PTPL1 fördert, bei der Herstellung eines Medikaments, das nützlich beim Fördern von Apoptose ist, zum Beispiel beim Behandeln von Krebs oder bei der Auflösung von Entzündung, wobei die Verbindung ein Molekül ist, das natives TAPP voller Länger kodiert.

## Revendications

1. Procédé pour identifier un composé permettant de moduler l'activité cellulaire ou la localisation de PTPL1, comprenant l'étape consistant à identifier un composé qui module l'interaction de PTPL1 avec TAPP ; ou qui module ou imite l'interaction de TAPP avec PtdIns(3,4)P₂ ; ou qui module la localisation cellulaire de TAPP.

2. Procédé selon la revendication 1, dans lequel le procédé comprend l'étape consistant à utiliser la modélisation moléculaire pour sélectionner ou concevoir un composé qui est apte à interagir avec le domaine de liaison aux phosphoinositides de TAPP, dans laquelle une structure tridimensionnelle d'au moins une partie du domaine de liaison aux phosphoinositides de TAPP est comparée à une structure tridimensionnelle d'un composé, et un composé qui est apte à interagir avec ledit domaine de liaison aux phosphoinositides est sélectionné.

3. Procédé selon la revendication 2, dans lequel la structure tridimensionnelle d'au moins une partie du domaine de liaison aux phosphoinositides de la TAPP est une structure tridimensionnelle d'au moins une partie du site de liaison aux phosphoinositides de la TAPP, et un composé qui est apte à interagir avec ledit site de liaison aux phosphoinositides est sélectionné.

4. Procédé selon la revendication 1, dans lequel le procédé comprend les étapes consistant à exposer un polypeptide TAPP ou PTPL1 à un composé à tester ; et à déterminer si le composé à tester module l'interaction de PTPL1 avec TAPP ; ou module ou imite l'interaction de TAPP avec PtdIns(3,4)P₂ ; ou module la localisation cellulaire de TAPP.

5. Procédé selon la revendication 1 ou 4, dans lequel le TAPP comprend le domaine PH C-terminal de la TAPP1 ou TAPP2 humaine et les trois ou quatre résidus les plus C-terminaux de la TAPP1 ou TAPP2 humaine de pleine longueur ; et dans lequel le PTPL1 comprend le domaine PDZ1 et/ou PDZ5 de la PTPL1 humaine et éventuellement également le domaine phosphatase de la PTPL1 humaine.

6. Procédé selon l'une quelconque des revendications 1, 4 ou 5, comprenant l'étape consistant à utiliser un système d'essai basé sur la fluorescence.

7. Procédé pour sélectionner un composé permettant de moduler une signalisation *via* PtdIns(3,4)P₂, le procédé comprenant l'étape consistant à identifier un composé qui module l'interaction entre TAPP et PTPL1.

8. Procédé selon la revendication 7, dans lequel le procédé comprend les étapes consistant à exposer un polypeptide TAPP ou PTPL1 à un composé à tester ; et à déterminer si le composé à tester module l'interaction entre TAPP et PTPL1.

9. Kit d'éléments utile pour réaliser un procédé de criblage de l'invention, comprenant TAPP ou un polynucléotide codant TAPP, et PTPL1 ou un polynucléotide codant PTPL1.

10. Procédé pour moduler l'activité cellulaire ou la localisation de PTPL1, le procédé comprenant l'étape consistant à exposer la PTPL1 à un composé qui module l'interaction de PTPL1 avec TAPP ; ou qui module ou imite l'interaction de TAPP avec PtdIns(3,4)P₂ ; ou qui module la localisation cellulaire de TAPP, dans lequel le procédé est réalisé *in vitro* et dans lequel le composé est un composé qui inhibe l'interaction de TAPP avec PTPL1, où le composé est un ARNsi, un ARNi, une molécule antisens ou un ribozyme dirigé(e) contre un polynucléotide codant pour TAPP ; ou dans lequel le composé est un composé qui favorise l'interaction de TAPP avec PTPL1, où le composé est une molécule codant une TAPP native de pleine longueur.

11. Procédé pour moduler une signalisation via PtdIns(3,4)P₂, le procédé comprenant l'étape consistant à exposer la PTPL1 à un composé qui module l'interaction entre TAPP et PTPL1, dans lequel le procédé est réalisé *in vitro* et dans lequel le composé est un composé qui inhibe l'interaction de TAPP avec PTPL1, où le composé est un ARNsi, un ARNi, une molécule antisens ou un ribozyme dirigé(e) contre un polynucléotide codant pour TAPP ; ou dans lequel le composé est un composé qui favorise l'interaction de TAPP avec PTPL1, où le composé est une molécule codant une TAPP native de pleine longueur.

12. Utilisation d'un composé qui inhibe l'interaction de TAPP avec PTPL1, pour la préparation d'un médicament destiné au traitement du diabète, à l'inhibition de l'apoptose, au traitement d'une maladie ischémique, à la guérison des plaies ou à la régénération nerveuse, dans laquelle le composé est un ARNsi, un ARNi, une molécule antisens ou un ribozyme dirigé(e) contre un polynucléotide codant TAPP.

13. Utilisation d'un composé qui favorise l'interaction de TAPP avec PTPL1, pour la préparation d'un médicament utile pour favoriser l'apoptose, par exemple dans le traitement d'un cancer ou dans la résolution d'une inflammation, dans laquelle le composé est une molécule codant une TAPP native de pleine longueur.
